# EUROPÄISCHE PATENTSCHRIFT

(11) **EP 2 298 916 B1**
(45) Veröffentlichungstag und Bekanntmachung des Hinweises auf die Patenterteilung: **18.03.2015**
(21) Anmeldenummer: 10012537.6
(22) Anmeldetag: 15.11.2001
(51) Int. Cl.: C12N 15/82, C12N 1/21, A01H 5/00, A01H 5/10

(54) **Gewebespezifische Promotoren aus der Zuckerrübe**
Tissue-specific promoters from sugar beet
Promoteurs spécifiques de tissu de betterave sucrière

(30) Priorität: 16.11.2000 EP 00124989
(43) Veröffentlichungstag der Anmeldung: 23.03.2011
(62) Teilanmeldung aus: 01994666.4
(73) Patentinhaber: KWS Saat AG, 37555 Einbeck (DE)
(72) Erfinder: Stahl, Dietmar Jürgen, 37574 Einbeck (DE); Hehl, Reinhard, 38114 Braunschweig (DE); Kloos, Dorothee U., 81479 München (DE)
(74) Vertreter: Westhoff, Markus

(56) Entgegenhaltungen:
- WO-A-97/32027
- WO-A1-98/08962
- WILLMOTT R L ET AL: "DNase1 footprints suggest the involvement of at least three types of transcription factors in the regulation of alpha-Amy2/A by gibberellin.", PLANT MOLECULAR BIOLOGY, Bd. 38, Nr. 5, November 1998 (1998-11), Seiten 817-825, XP002181270, ISSN: 0167-4412 -& DATABASE EM_PLN EMBL Heidelberg, Germany; AC: AJ010728 3. September 1998 (1998-09-03), LAZARUS C M ET AL.: XP002181271,

## Beschreibung

Die vorliegende Erfindung betrifft Promotoren und ihre Verwendung sowie transgene Pflanzen.

Durch molekularbiologische Methoden ist es möglich, Nutzpflanzen genetisch zu verändern und Proteine gezielt zu exprimieren. Hierbei kommt der Auswahl eines geeigneten Promotors eine erhebliche Bedeutung zu. Infolgedessen existiert ein großes Bedürfnis nach gut charakterisierten Promotoren mit spezifischen Eigenschaften.

Eine große Zahl pflanzlicher Promotoren konnte in den letzten Jahren isoliert und auf ihre Wirkung hin untersucht werden. Breite Anwendung haben inzwischen die aus *Agrobacterium tumefaciens* isolierten Octopinsynthase (ocs), Nopalinsynthase (nos) und Mannopinsynthase (mas) bzw. TR-Promotoren (De Greve et al., 1982, Depicker et al., 1982; Velten et al., 1984) und der 35S-Promotor des Blumenkohlmosaikvirus (Odell et al., 1985) gefunden. Pflanzliche Promotoren mit einer konstitutiven Aktivität sind für Tabak (WO 97/28268) und Himbeere (WO 97/27307) beschrieben worden.

Organ-, gewebe- oder zellspezifische Promotoren können für die Expression von Genen in spezifischen Pflanzenteilen verwendet werden. Spezifität kann in diesem Zusammenhang bedeuten, daß ein Promotor hauptsächlich oder ausschließlich in einem Organ, Gewebe oder Zelltyp aktiv ist. Hauptsächlich in einem bestimmten Organ aktiv sind z.B. die Tomatenpromotoren TFM7 und TFM9 in Tomatenfrüchten (US 5,608,150), ein Rapspromotor in Wurzeln (WO 94/02619), ein Sonnenblumenpromotor in Samen (WO 98/45460) und ein Kartoffelpromotor (WO 98/18940) in Blättern. Diese genannten Promotoren zeigen ihre höchste Aktivität in den erwähnten Organen. Eine exklusive, ausschließliche Aktivität für ein bestimmtes Kompartiment wurde für einen schließzellspezifischen Promotor aus der Kartoffel (DE 42 07358 A1), für den tapetum-spezifischen Promotor TA29 aus Tabak (EP 0344 029 B1) und für den pistil- und pollen-spezifischen SLG Promotor aus Brassica (Dzelzkàlns et al., 1993) beschrieben.

Aus der Zuckerrübe ist ein organspezifischer Promotor bekannt, der hauptsächlich im Speicherwurzelgewebe der Zuckerrübe aktiv ist (WO 97/32027). Allerdings ist dieser Promotor des Sucrosesynthase-Gens nicht nur in Wurzeln, sondern auch mit geringer Aktivität in anderen Geweben wie Blättern aktiv (Hesse and Willmitzer, 1996).

Aufgabe der vorliegenden Erfindung ist es daher, neue Promotoren und Pflanzen bereitzustellen mit der Möglichkeit zur gewebespezifischen Expression von Genen entweder in Wurzeln oder oberirdischen Pflanzenteilen.

Erfindungsgemäß erfolgt die Lösung der gestellten Aufgabe durch die Promotoren gemäß dem Hauptanspruch sowie einer transgenen Pflanze erhältlich durch Transformation einer Pflanzenzelle mit einem Promotor nach dem Hauptanspruch, der mit einem übertragenen Gen operativ verbunden ist, und anschließender Regeneration der transgenen Pflanze.

Einige der in dieser Anmeldung verwendeten Begriffe werden nachfolgend näher erläutert:
Unter einem Promotor wird eine Nukleinsäuresequenz verstanden, die die Expression eines Gens unter ihrer Kontrolle gegebenenfalls in Abhängigkeit von endogenen und exogenen Faktoren steuert. Zu diesen Faktoren gehören z.B. Induktoren, Repressoren und ähnliche DNA-bindende Proteine, als auch Umwelteinflüsse. Ein Promotor kann aus mehreren Elementen bestehen. Er umfaßt jedoch mindestens ein regulatorisches Element, das für die Transkription des unter seiner Kontrolle stehenden Gens zuständig ist.

Ein in oberirdischen und Chloroplasten enthaltenden Pflanzenteilen, wie Blättern, und nicht in unterirdischen Organen von Pflanzen aktiver Promotor zeigt in Blättern eine durch RNA-Blots meßbare Aktivität, die bei vergleichbaren Versuchsbedingungen in unterirdischen Organen von Pflanzen zu weniger als 20 %, vorzugsweise zu weniger als 10% und insbesondere zu weniger als 5% nachweisbar ist. Diese Spezifität beschränkt sich nicht auf einen bestimmten Untersuchungszeitpunkt, sondern ist grundsätzlich während der gesamten Vegetationszeit gegeben.

Ein in unterirdischen Organen und nicht in oberirdischen Organen von Pflanzen aktiver Promotor zeigt beispielsweise in Wurzeln eine durch RNA-Blots meßbare Aktivität, die bei vergleichbaren Versuchsbedingungen in oberirdischen Organen von Pflanzen wie Petiolen, Blättern und Blüten zu weniger als 20 %, vorzugsweise zu weniger als 10% und insbesondere zu weniger als 5% nachweisbar ist. Diese Spezifität beschränkt sich nicht auf einen bestimmten Untersuchungszeitpunkt, sondern ist grundsätzlich während der gesamten Vegetationszeit gegeben.

"Derivate" eines Promotors sind verkürzte oder verlängerte oder abschnittsweise identische Versionen dieses Promotors oder Homologe mit gleichen, modifizierten oder singulären Eigenschaften.

"Pathogeninduzierbarkeit" bedeutet das Einwirken von äußeren Faktoren auf eine Pflanze, die eine Abwehrreaktion derselben nach sich zieht. Dabei kann es sich um Angriffe durch Insekten (Fraß), Bakterien, Pilze, Nematoden oder andere Pathogene handeln, aber auch um abiotische Einwirkungen, wie mechanische Verwundungen (z.B. durch Hagelschlag).

"Direkte antifungale Wirkung" bedeutet, daß Genprodukte unmittelbar antifungal wirken, indem sie z.B. Zellwände auflösen oder für Phytoalexinsynthasen codieren bzw. für Metabolite, die hemmend in den pilzlichen Stoffwechsel eingreifen.

"Indirekte antifungale Wirkung" bedeutet, daß Genprodukte die pflanzliche Genabwehr aktivieren. Zu diesen Genen gehören z.B. Resistenzgene, Komponenten der Signaltransduktion (wie Kinasen, Phosphatasen), Transkriptionsfaktoren oder Enzyme, die Signalsubstanzen produzieren (wie Ethylenbildende, Salicylsäurebildende oder Jasmonatbildende Enzyme, reaktive Sauerstoffspezies bildende Enzyme, Stickstoffmonoxydbildende Enzyme).

Als "sink"-Blätter werden hier solche Blätter bezeichnet, die aufgrund ihrer geringen Größe mehr Kohlenhydrate verbrauchen als sie selber produzieren.

"source"-Bätter sind Blätter, die aufgrund ihrer Größe mehr Kohlenhydrate produzieren als sie selber verbrauchen.

Unter "Infektion" ist der früheste Zeitpunkt zu verstehen, bei dem der Stoffwechsel des Pilzes (bzw. das Wachstum des Pilzes) auf eine Penetration des Wirtsgewebes vorbereitet wird. Dazu gehören z.B. das Auswachsen von Hyphen oder die Bildung von spezifischen Infektionsstrukturen wie Penetrationshyphen und Appressorien.

Der Ausdruck "Homologie" bedeutet hierbei eine Homologie von mindestens 70 % auf DNA-Ebene, die gemäß bekannter Verfahren, z.B. der computergestützten Sequenzvergleiche (S.F. Altschul et al. (1990), Basic Local Alignment search tool, J.Mol. Biol. 215: 403-410) bestimmt werden kann.

Komplementäre Nukleotidsequenz bedeutet bezogen auf eine doppelsträngige DNA, daß der zum ersten DNA Strang komplementäre zweite DNA Strang entsprechend den Basenpaarungsregeln die Nukleotidbasen aufweist, die zu den Basen des ersten Stranges korrspondieren.

Der hier verwendete Begriff "hybridisieren" bedeutet hybridisieren unter üblichen Bedingungen, wie sie in Sambrook et al. (Molecular Cloning. A laboratory manual, Cold Spring Harbor Laboratory Press, 2. Aufl. 1989) beschrieben sind, bevorzugt unter stringenten Bedingungen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Wenig stringente Hybridisierungsbedingungen sind beispielsweise: Hybridisieren in 4 x SSC bei 37 °C und anschließendes mehrfaches Waschen in 1 x SSC bei Raumtemperatur. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C.

Die Erfindung wird nachfolgend und mit Bezug auf die Figuren und Beispiele näher erläutert.

Die erfindungsgemäßen Promotoren zeichnen sich besonders dadurch aus, daß sie ausschließlich in oberirdischen Organen einer Pflanze aktiv sind. Mit ihnen lassen sich transgene Pflanzen mit besonderen Eigenschaften herstellen. In bevorzugter Weise lassen sie sich zu folgenden Zwecken verwenden:
a. Veränderung des Kohlenhydratmetabolismus
b. Vermeidung von Speichersubstanzverlusten
c. Expression eines Invertaseinhibitors
d. Expression einer Fructosyltransferase
e. Expression einer Levansucrase
f. Expression von Genen codierend für Transporterproteine für N-Verbindungen
g. Ausprägung von Merkmalen, die die Resistenz/Toleranz gegenüber Pathogenen erhöhen

Die Promotoren gemäß SEQ ID NO: 1 und SEQ ID NO: 2 sind in Wurzeln, insbesondere der Zuckerrübe, aktiv, nicht dagegen in oberirdischen Organen der betreffenden Pflanze. Diese Eigenschaft kann zur Verbesserung des Stoffwechsels der transgenen Pflanzen, speziell des Kohlenhydratstoffwechsels von Zuckerrüben, genutzt werden. Eine Verbesserung des Kohlenhydratstoffwechsels besteht darin, Verluste an Sucrose und die Akkumulation von Glucose und Fructose während der Lagerung der Rübenkörper nach der Ernte zu reduzieren. Durch Verwendung eines Invertaseinhibitorgens unter der Expressionskontrolle von SEQ ID NO: 1 und SEQ ID NO: 2 kann die Aktivität der vakuolären Invertase in der Wurzel reduziert werden. Durch die organspezifische Expression des Inhibitors werden pleiotrope Effekte, die eine Hemmung der Invertase in der gesamten Pflanze mit sich bringt, vermieden.

Weitere Verbesserungen des Kohlenhydratstoffwechsels sind die Produktion des Süßstoffes Palatinit oder die Synthese von Polyfructanen in der Wurzel von Zuckerrüben unter der Verwendung der beschriebenen Sequenzen.

Die wurzelspezifisch aktiven Promotoren (SEQ ID NO: 1 und SEQ ID NO: 2) können auch genutzt werden, um den Stickstoffstoffwechsel der Pflanzen zu verbessern. Dazu werden Transportproteingene für Ammonium- (NH₄⁺), Nitrat- (NO₃⁻) und Nitrit (NO₂⁻)-Ionen in der Wurzel überexprimiert und die Aufnahme der genannten Ionen verstärkt. Eine weitere Verbesserung des N-Stoffwechsels stellt die reduzierte Einlagerung von "schädlichem Stickstoff' in die Speicherorgane der Pflanze dar. Erhöhte Konzentrationen an N-Verbindungen in Speicherorganen reduzieren oft den ernährungsphysiologischen Wert von Ernteprodukten oder Erschweren die Isolierung von Speicherstoffen wie Sucrose aus Zuckerrübenwurzeln. Eine reduzierte Einlagerung von "schädlichem Stickstoff' in die Wurzel kann durch eine verminderte Aufnahme von Ammonium- und Nitrationen aus dem Boden erreicht werden. Zu diesem Zweck werden die wurzelspezifisch aktiven Promotoren genutzt, um z. B. durch einen "antisense"-Ansatz die Expression endogener Transportergene organspezifisch zu reduzieren.

Die erfindungsgemäßen Promotoren können auch genutzt werden, um die Krankheitsresistenz der Pflanzen zu verbessern.

Virale Infektionen der Zuckerrübe sind oft nur auf ein Organ wie die Wurzel oder die oberirdischen Pflanzenteile beschränkt. So infiziert und koloniert das Virus BNYW in erster Linie die Rübenwurzel und die Vergilbungsviren BMYV und BYV werden nur in Blättern gefunden. Die wurzelaktiven Promotoren und die nur in oberirdischen Organen aktiven Promotoren können genutzt werden, um die auf dem gene silencing bzw. der antisense-Technik beruhenden Virusresistenzkonzepte organspezifisch umzusetzen.

### Sequenzen und Abbildungen

Die Nukleotidsequenzen der SEQ ID NO: 1, SEQ ID NO: 2, SEQ ID NO: 3 und SEQ ID NO: 4 sind in 5'- 3'- Orientierung dargestellt.

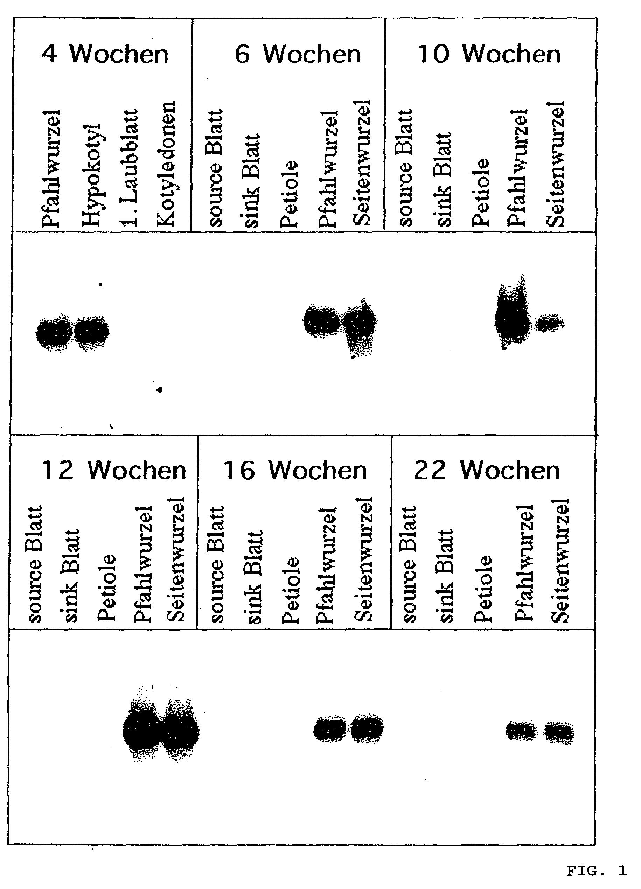

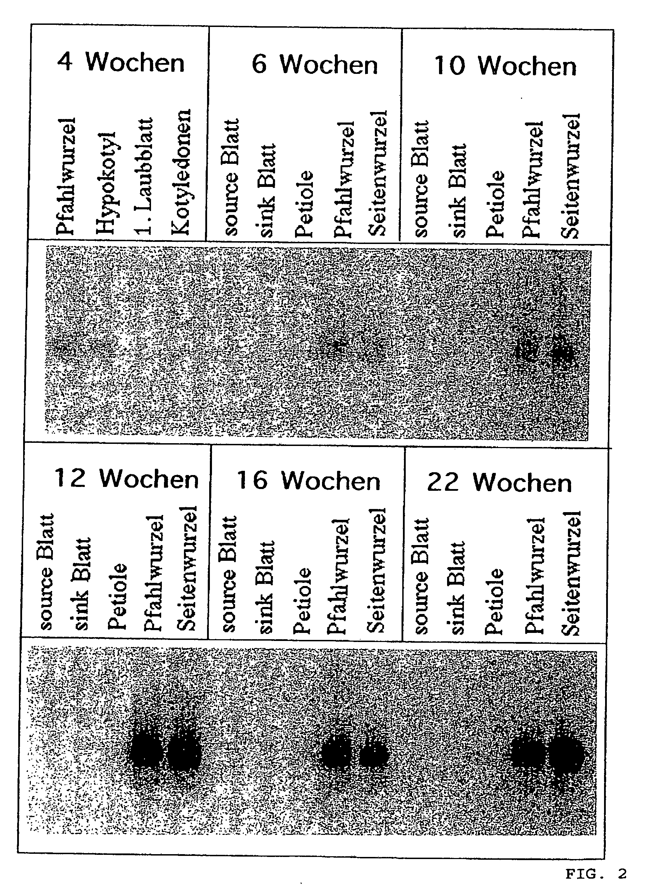

Abbildungen 1 und 2 zeigen die ausschließlich wurzelspezifische Expression der Gene 2-1-48 und 2-1-36 während der Rübenentwicklung durch ein RNA-Blot Experiment. Jeweils 10 µg Gesamtzell-RNA, die aus verschiedenen Organen von 4 , 6, 10, 12, 16 und 22 Wochen alten Zuckerrüben isoliert worden waren, wurden in einem denaturierenden Formaldehyd-Agarosegel aufgetrennt. RNA wurde aus der Pflahlwurzel, den Seitenwurzeln, "sink"- und "source" Blättern sowie im Fall der 4 Wochen alten Pflanzen aus dem Hypokotyl und den Keimblättern isoliert. Das jeweilige cDNA Fragment 2-1-48 bzw. 2-1-36 wurde als Hybridisierungssonde verwendet.

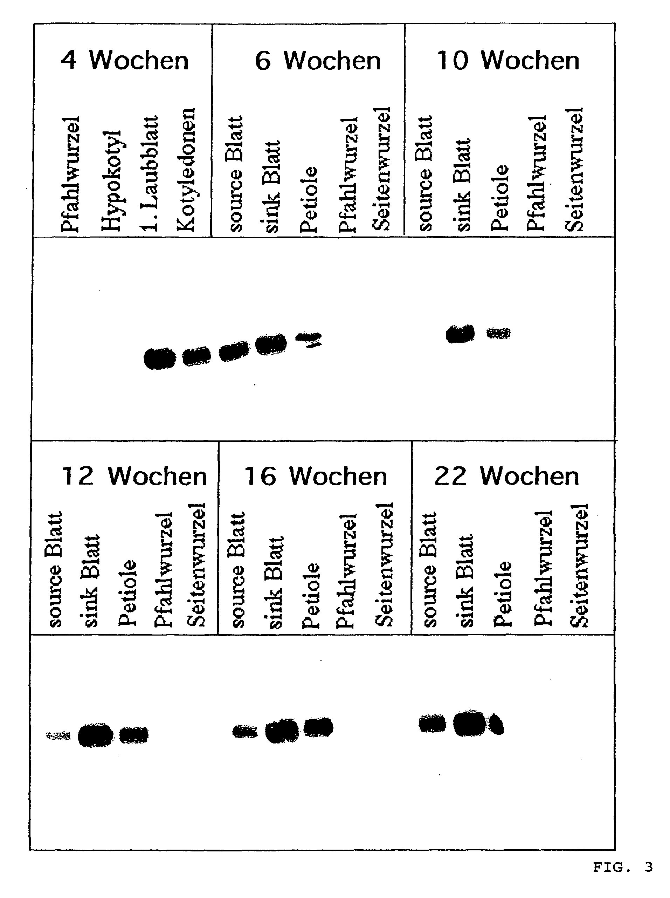

Abbildung 3 zeigt durch ein RNA-Blot Experiment, daß das Gens 2-3-9 während der Rübenentwicklung ausschließlich in den oberirdischen Pflanzenorganen exprimiert wird. Jeweils 10 µg Gesamtzell-RNA, die aus verschiedenen Organen von 4 , 6, 10, 12, 16 und 22 Wochen alten Zuckerrüben isoliert worden waren, wurden in einem denaturierenden Formaldehyd-Agarosegel aufgetrennt. RNA wurde aus der Pflahlwurzel, den Seitenwurzeln, "sink"- und "source" Blättern sowie im Fall der 4 Wochen alten Pflanzen aus dem Hypokotyl und den Keimblättern isoliert. Das cDNA Fragment 2-3-9 wurde als Hybridisierungssonde verwendet.

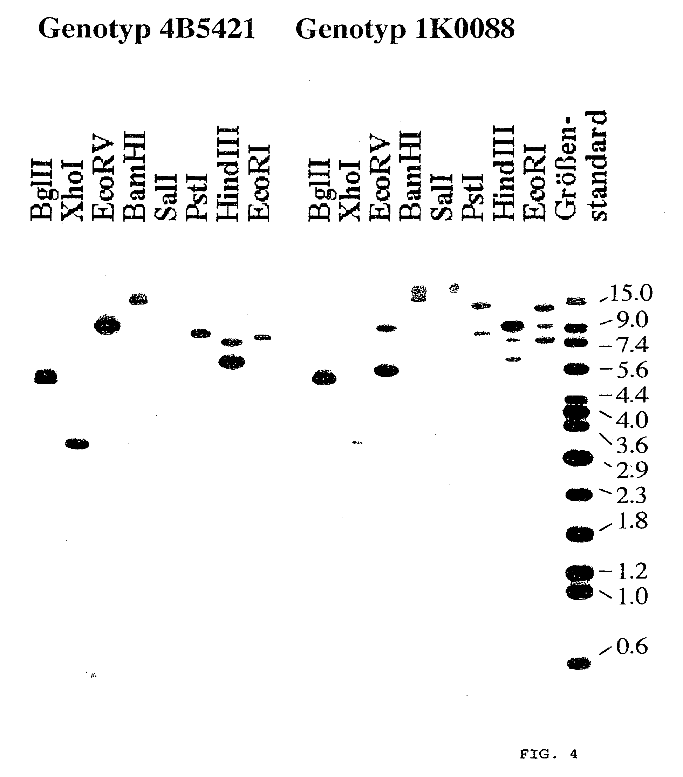

Abbildung 4 zeigt durch ein DNA-Blot Experiment, daß in dem Genom des Zuckerrübengenotypen 1 K0088 zwei Kopien des Gens 2-3-9 und in dem Genotyp 4B5421 nur eine Genkopie vorliegt. Jeweils 10 µg genomische DNA wurden pro Restriktionsverdau verwendet. Das cDNA Fragment 2-3-9 wurde als Hybridisierungssonde verwendet.

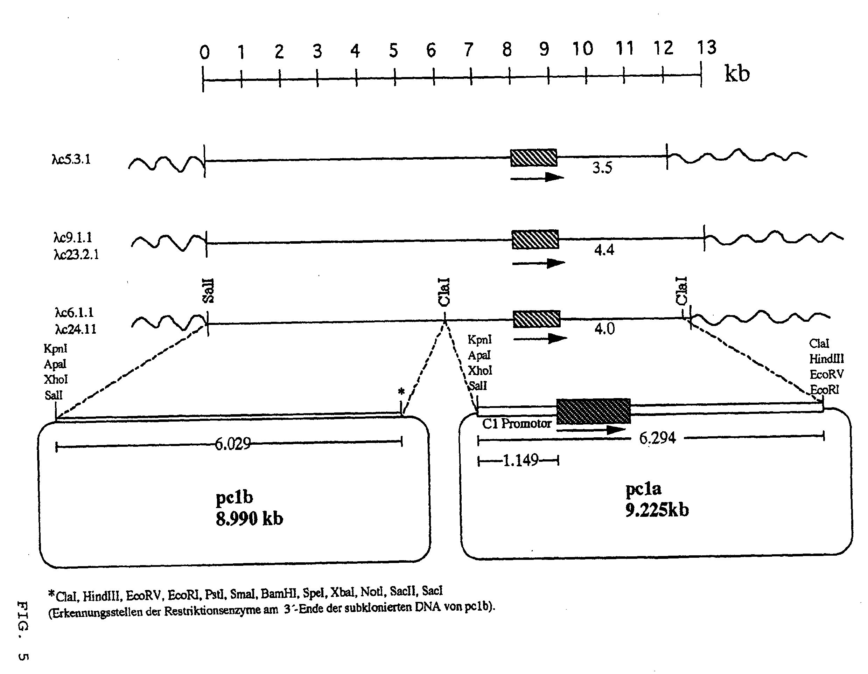

Abbildung 5 zeigt auf der Grundlage der durchgeführten Restriktionsanalysen die Lage und Orientierung des codierenden Bereichs der 1. Kopie des Gens 2-3-9 und des C1 Promotors für 5 der isolierten Lambda-Phagen. Weiterhin ist die Subklonierung des Inserts des Phagen λ6.1.1 in die Plasmide pc1a und pc1 b dargestellt. Das gestreifte Kästchen stellt den codierenden Bereich der 1. Kopie des Gens 2-3-9 dar.

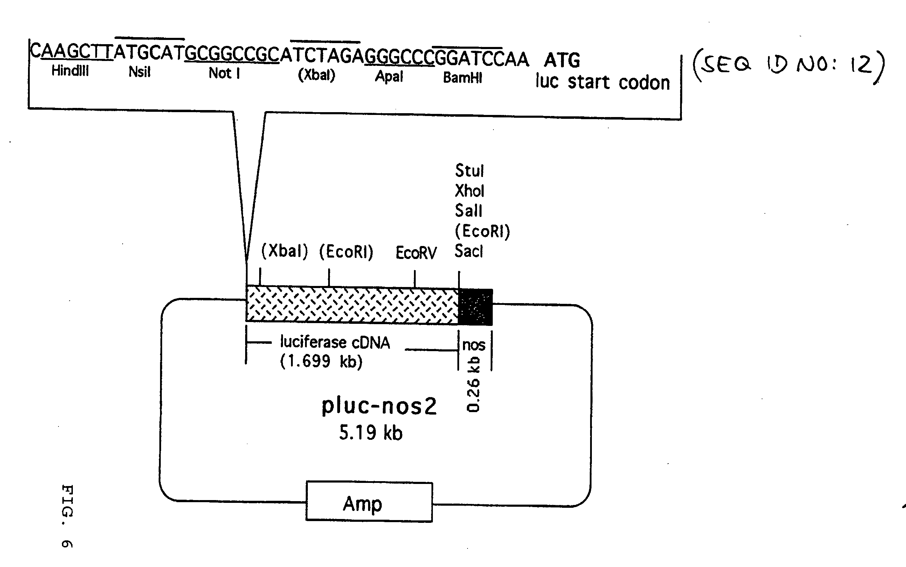

Abbildung 6 zeigt den 5.19 kb großen Reportergenvektor pluc-nos2. Das Plasmid pluc-nos2 enthält das Luciferasegen aus *Photinus pyralis* und den nos-Terminator. Die multiple Klonierungsstelle im 5'-Bereich des Reportergens erlaubt die Insertion von Promotorfragmenten. Die eingeklammerten Restriktionsenzyme schneiden in dem Plasmid mehrmals.

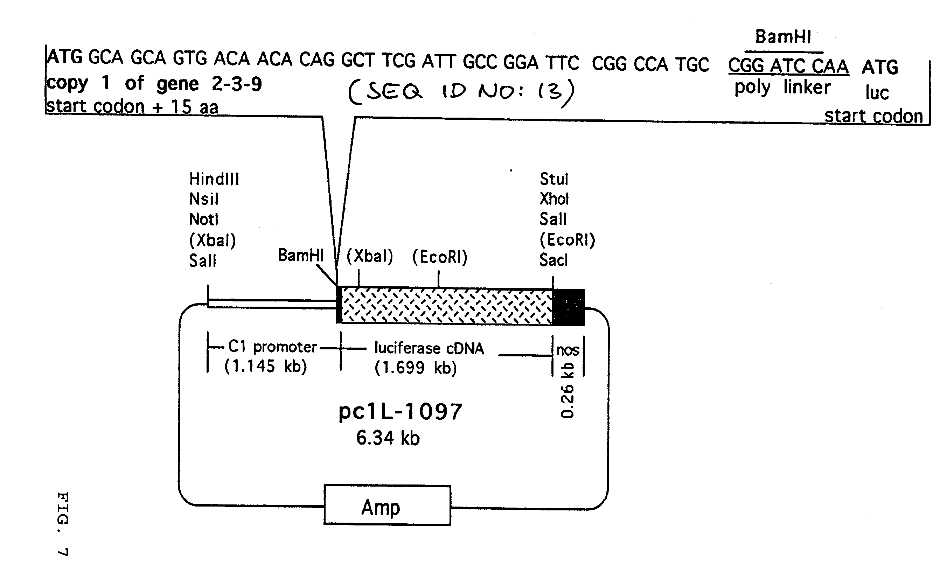

Abbildung 7 zeigt das 6.34 kb große Reportergenkonstrukt pc1 L-1097. Der Vektor pc1 L-1097 entstand durch Insertion des C1 Promotorfragmentes (Position 1-1145 der SEQ ID NO:3) in den Vektor pluc-nos2. Die eingeklammerten Restriktionsenzyme schneiden in dem Plasmid mehrmals.

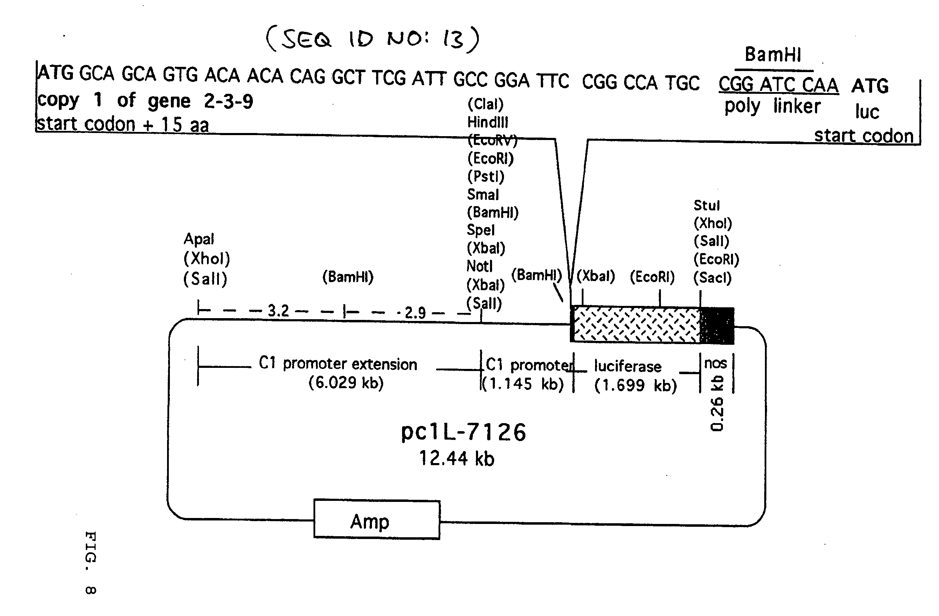

Abbildung 8 zeigt das 12.44 kb große Reportergenkonstrukt pc1 L-7126. Nach Isolierung des 6029 bp großen genomischen 5'-Bereichs der Kopie 1 des Gens 2-3-9 aus dem Vektor pc1b wurde das DNA Fragment in den Vektor pc1 L-1097 inseriert. Der resultierende Vektor pc1 L-7126 umfaßt den regulatorischen 5'-Bereich der 1. Kopie des Gens 2-3-9 von Position 1-7126. Die eingeklammerten Restriktionsenzyme schneiden in dem Plasmid mehrmals.

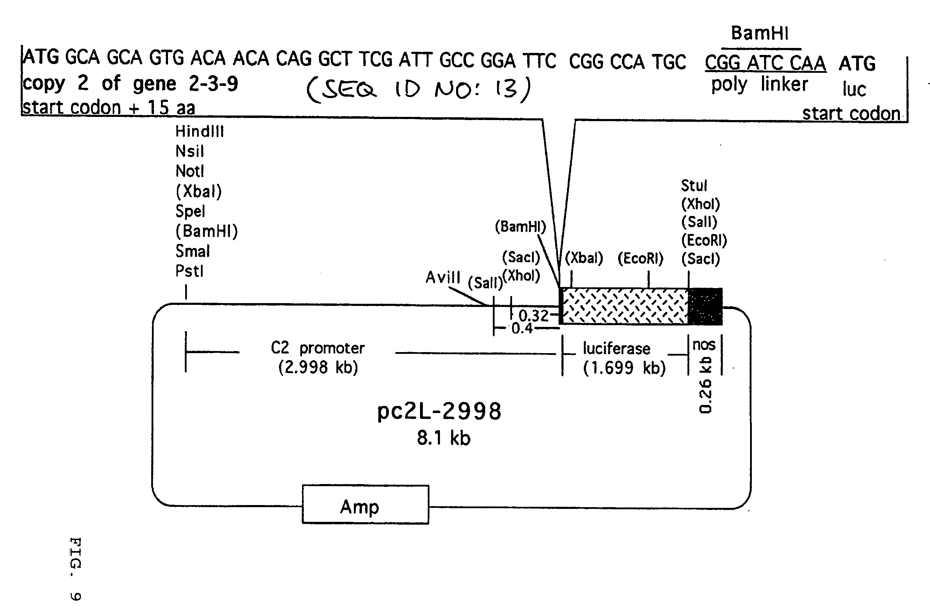

Abbildung 9 zeigt das 8.1 kb große Reportergenkonstrukt pc2L-2998. Der Vektor pc2L-2998 entstand durch Insertion des C2 Promotorfragmentes (Position 1-3046 der Nukleotidsequenz der SEQ ID NO: 4) in den Vektor pluc-nos2. Die eingeklammerten Restriktionsenzyme schneiden in dem Plasmid mehrmals.

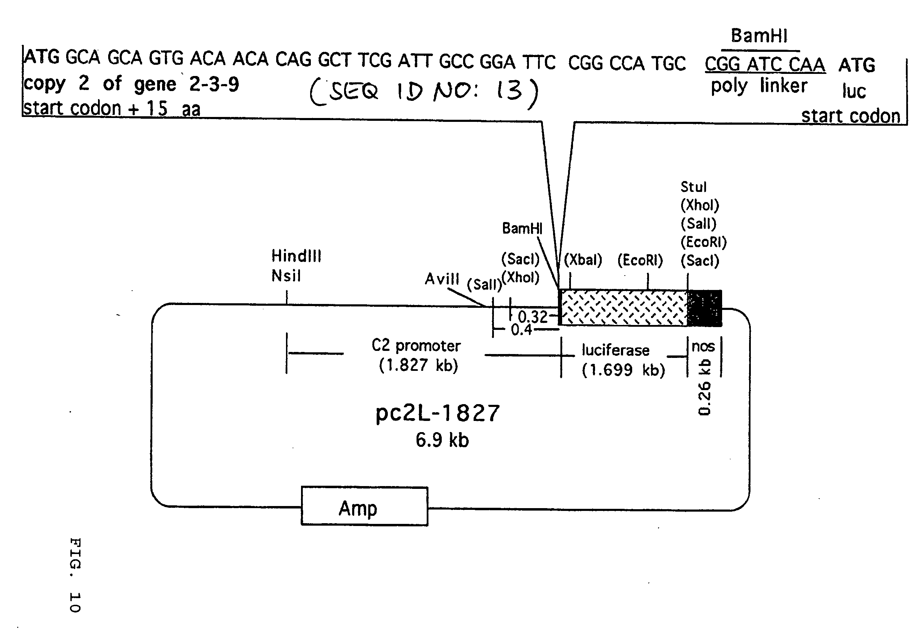

Abbildung 10 zeigt das 6.9 kb große Reportergenkonstrukt pc2L-1827. Der Vektor pc2L-1827 entstand durch eine 5'-Deletion des C2 Promotors des Plasmides pc2L-2998. Der C2 Promoter in dem Vektor pc2L-1827 umfaßt die Nukleotidpositionen 1172-3046 der Nukleotidsequenz SEQ ID NO: 4. Die eingeklammerten Restriktionsenzyme schneiden in dem Plasmid mehrmals.

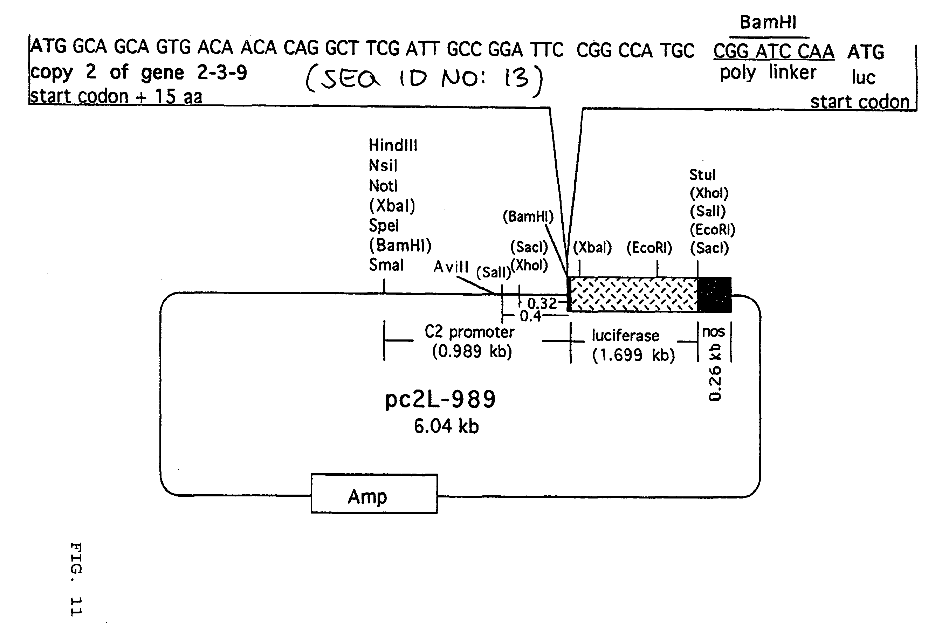

Abbildung 11 zeigt das 6.04 kb große Reportergenkonstrukt pc2L-989. Der Vektor pc2L-989 entstand durch eine 5'-Deletion des C2-Promotors des Plasmides pc2L-2998. Der C2-Promoter in dem Vektor pc2L-989 umfaßt die Nukleotidpositionen 2011-3046 der Nukleotidsequenz der SEQ ID NO: 4. Die eingeklammerten Restriktionsenzyme schneiden in dem Plasmid mehrmals.

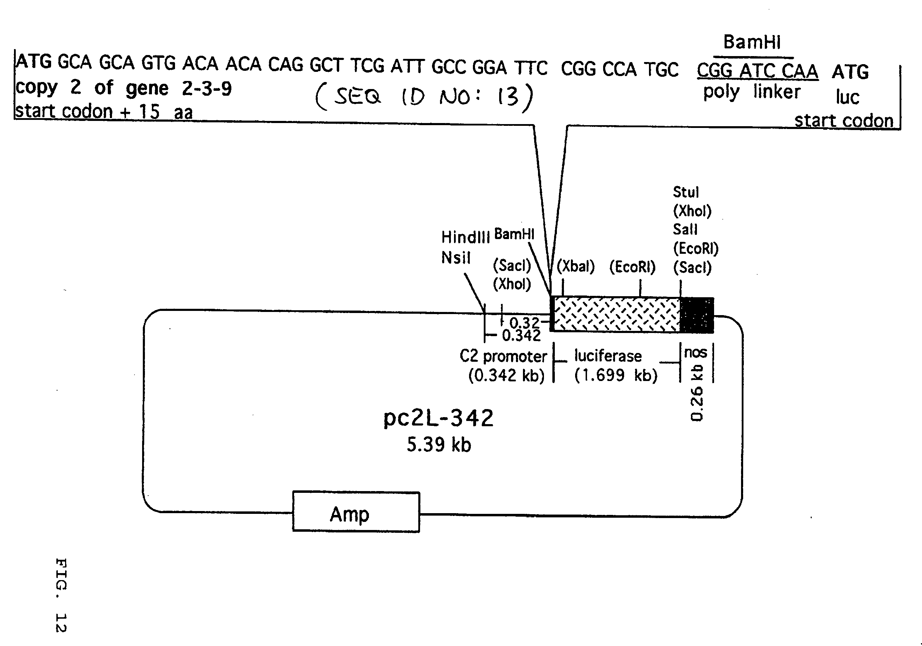

Abbildung 12 zeigt das 5.39 kb große Reportergenkonstrukt pc2L-342. Der Vektor pc2L-342 entstand durch eine 5'-Deletion des C2-Promotors des Plasmides pc2L-2998. Der C2-Promoter in dem Vektor pc2L-342 umfaßt die Nukleotidpositionen 2657-3046 der Nukleotidsequenz der SEQ ID NO: 4. Die eingeklammerten Restriktionsenzyme schneiden in dem Plasmid mehrmals.

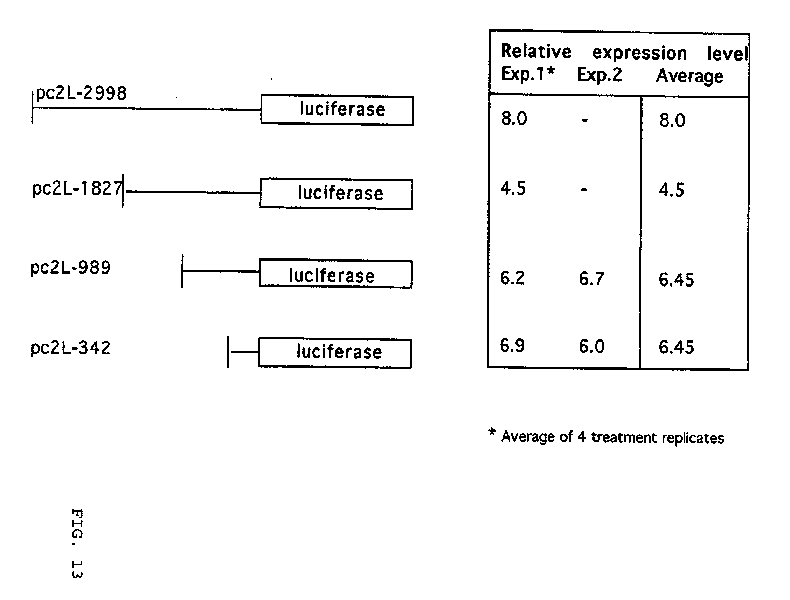

Abbildung 13 zeigt die Aktivität der Reportergenkonstrukte pc2L-2998, pc2L-1827, pc2L-989 und pc2L-342 nach ballistischer Transformation in Zuckerrüben blätter. Pro Konstrukt wurde 1-2 DNA Präparationen mit jeweils 4 Versuchswiederholungen verwendet. Die ermittelten *Photinus pyralis* Luciferaseaktivitäten wurden durch parallele Messung der *Renilla reniformis* Luciferaseaktivität normalisiert und Schwankungen in der Transformationseffizienz dadurch kompensiert.

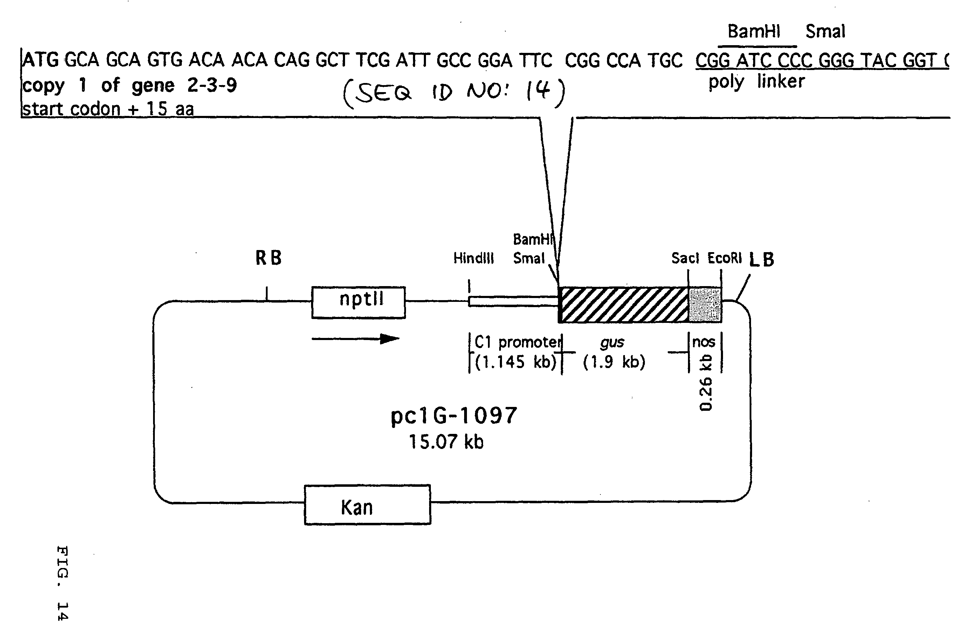

Abbildung 14 zeigt den 15.07 kb großen binären Pflanzentransformationsvektor pc1 G-1097. Der C1 Promotor ist translationell mit dem gus-Reportergen fusioniert. Der C1 Promotor umfaßt die Nukleotidpositionen 1-1145 der Nukleotidsequenz der SEQ ID NO: 3.

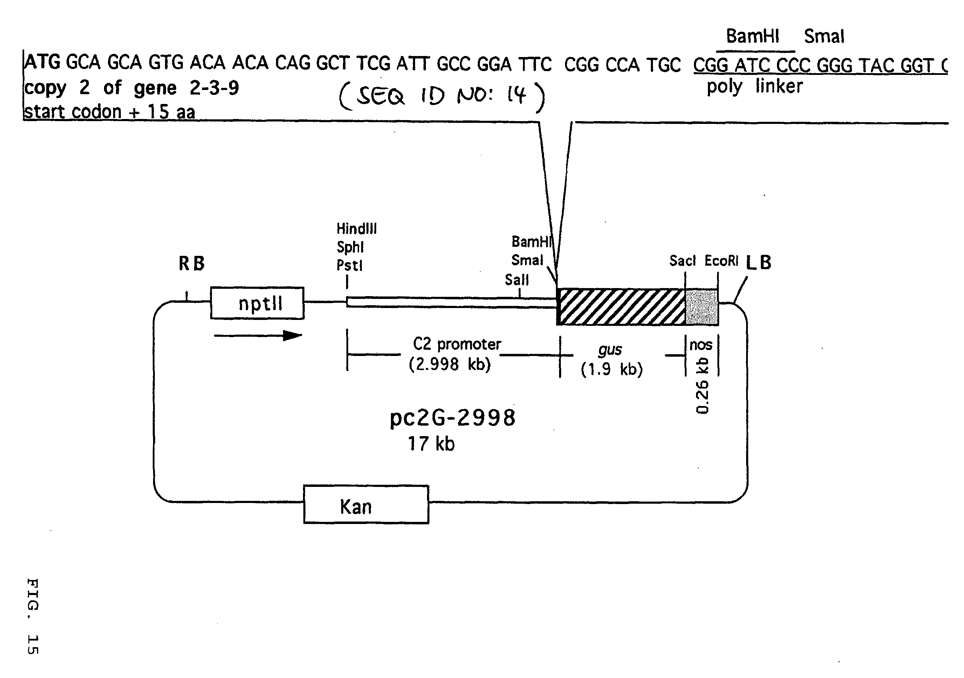

Abbildung 15 zeigt den 17 kb großen binären Pflanzentransformationsvektor pc2G-2998. Der C2 Promotor ist translationell mit dem *gus-*Reportergen fusioniert. Der C2 Promoter umfaßt die Nukleotidpositionen 1-3046 der Nukleotidsequenz der SEQ ID NO: 4.

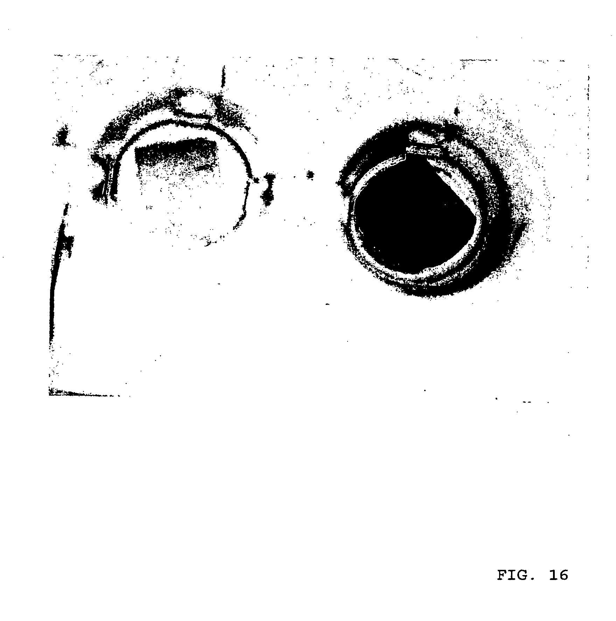

Abbildung 16 zeigt den histochemischen Nachweis der Aktivität des C1 Promotors in Blättern transgener Rapspflanzen. Blattstückchen der Transformanten pc1 G-1 097-86 (rechts) lassen sich aufgrund der *gus*-Reporteregenaktivität im Vergleich zu der nichttransgenen Kontrolle (links) blau färben.

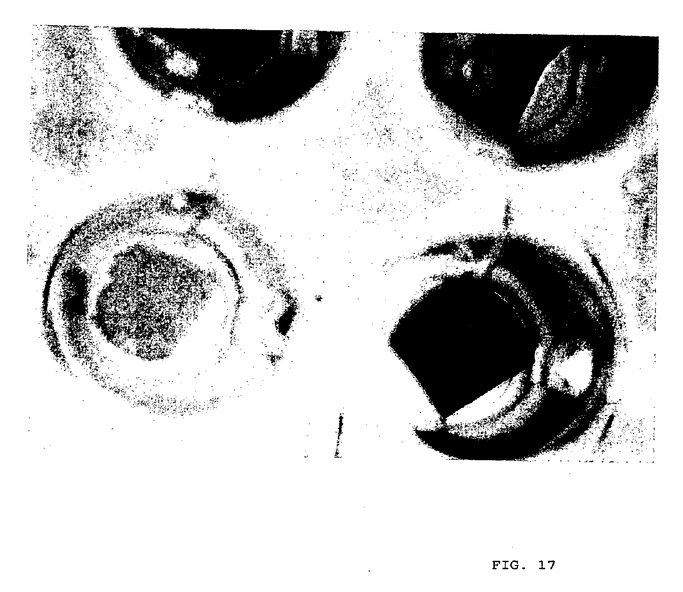

Abbildung 17 zeigt den histochemischen Nachweis der Aktivität des C1 Promotors in Blättern transgener Tabakpflanzen. Ein Blattstückchen der Transformante pc1G-1097-3 (rechts) wird aufgrund der *gus*-Reportergenaktivität im Vergleich zu der nicht-transgenen Kontrolle (links) blau gefärbt.

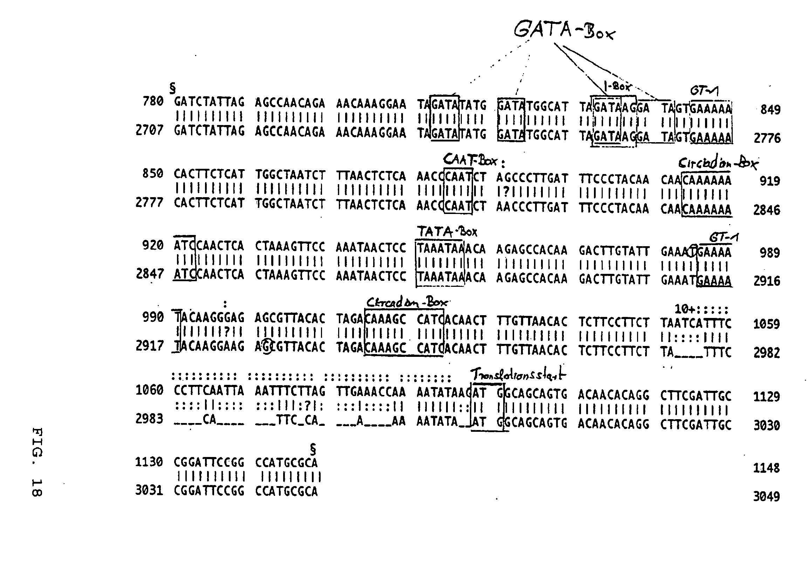

Abbildung 18 zeigt einen DNA-Sequenzvergleich zwischen den konservierten Bereichen des C1 und des C2 Promotors. Gekennzeichnet sind in der Abbildung die Positionen der GATA-Boxen, der 1-Box, der GT-1 Bindungsstellen, der CAAT-Box, zweier circadianer Boxen und der TATA-Boxen. Der Translationsstart der 1. Kopie des Gens 2-3-9 liegt bei Position 1098 und der Translationsstart der 2. Kopie des Gens 2-3-9 befindet sich bei Position 2998. Der Transkriptionsstartpunkt innerhalb des C1 Promotors liegt bei Position 984 und der Transkriptionsstartpunkt innerhalb des C2 Promotors befindet sich bei Position 2928.

### Beispiele

### Isolierung von wurzel- und blattspezifischen exprimierten cDNAs der Zuckerrübe

Mit der Methode der "Suppression Subtractive Hybridization" (Diatchenko et al., 1996) wurde eine Anreicherung von cDNA Fragmenten Blatt- bzw. Pfahlwurzel-exprimierter Gene der Zuckerrübe durchgeführt. Dazu wurde aus beiden Geweben zuerst Gesamt-RNA und anschließend polyA(+) RNA isoliert. Zusätzlich wurden Gesamt-RNAs aus Sprossen und Infloreszenzen isoliert, die für Northern-Blot Analysen eingesetzt wurden. Die folgenden Arbeiten wurden nach dem Protokoll des PCR-Select Systems der Firma CLONTECH durchgeführt. Mit der polyA(+) RNA aus Blatt und Pfahlwurzel wurde cDNA synthetisiert. Mit beiden cDNAs wurden zwei Subtraktionen durchgeführt. Zur Anreicherung von Blatt-spezifischen Genen wurde die Blatt cDNA als Tester gegen die cDNA aus der Pfahlwurzel subtrahiert. Zur Anreicherung von Pfahlwurzel-spezifischen Genen wurde die Pfahlwurzel cDNA als Tester gegen die cDNA aus dem Blatt subtrahiert. Bei der Subtraktion wurde genau nach dem Protokoll des CLONTECH-Kits vorgegangen, wobei auch die interne Kontrolle mit durchgeführt wurde. Alle weiteren molekularbiologischen Arbeiten wurden nach Standardprotokollen durchgeführt (Sambrook et al., 1989). Nach der Subtraktion lagen amplifizierte cDNA Fragmente vor, die entweder auf Blatt- oder Wurzel-spezifische Gene angereichert wurden.

Zur weiteren Untersuchung wurden die cDNA Fragmente in den TA Klonierungsvektor pCR2.1 (Invitrogen) kloniert und in *E. coli* transformiert. Eine Blau-Weiß Selektion ermöglichte die Identifizierung rekombinanter Plasmide (Sambrook et al., 1989). Bei den weißen Kolonien ist durch ein Insert die Expression der β-Galaktosidase unterdrückt, was zu weißen Kolonien führt, weil das dem Medium zugefügte Enzymsubstrat nicht mehr gespalten wird. Pro µg PCR Produkt wurden ca. 300 weiße Kolonien erhalten. Es wurden insgesamt 62 Klone von der Subtraktion auf Pfahlwurzel-spezifische Gene und 60 Klone der Subtraktion auf Blattspezifische Gene näher untersucht. Die DNA der Klone wurde dazu mit dem Restriktionsenzym *Rsa*I gespalten. Das befreit die Inserts von den Adaptoren. Dann wurde jeweils die Hälfte der gespaltenen DNA auf zwei Gelen elektrophoretisch aufgetrennt und die DNA auf Nylonmembranen transferiert. Zur Identifizierung von DNA Fragmenten, die spezifisch angereichert wurden, wurden die Filter mit den PCR Produkten (cDNA Fragmente) der beiden Subtraktionen hybridisiert. cDNA Fragmente, die mit den cDNA Fragmenten hybridisieren aus denen sie subkloniert wurden, aber nicht oder nicht so stark mit den cDNA Fragmenten der anderen Subtraktion wurden weiter analysiert, da sie potentiell angereicherte bzw. gewebespezifische Gene repräsentieren.

Unter den 62 Klonen aus der Subtraktion auf Pfahlwurzel-spezifische Gene wurden 18 identifiziert, die stark mit den cDNA Fragmenten der Subtraktion auf Pfahlwurzel-spezifische Gene und nicht oder nur sehr schwach mit den cDNA Fragmenten der Subtraktion auf Blatt-spezifische Gene hybridisieren. Unter den 60 Klonen von der Subtraktion auf Blatt-spezifische Gene wurden 23 identifiziert, die stark mit den cDNA Fragmenten der Subtraktion auf Blatt-spezifische Gene und nicht oder nur sehr schwach mit den cDNA Fragmenten der Subtraktion auf Pfahlwurzel-spezifische Gene hybridisieren. Alle so identifizierten cDNA Inserts beider Subtraktionen wurden durchsequenziert und mit einem Sequenzanalyseprogramm (Pileup, GCG Wisconsin Analysis Package) untereinander verglichen. Aufgrund von Ähnlichkeiten (Homologien) der Sequenzen untereinander blieben insgesamt 9 unterschiedliche cDNA Fragmente von der Subtraktion auf Pfahlwurzel-spezifische Gene und 14 unterschiedliche cDNA Fragmente von der Subtraktion auf Blatt-spezifische Gene übrig.

Alle 9 unterschiedlichen cDNA Fragmente aus der Subtraktion auf Pfahlwurzel-spezifische Gene und 7 der 14 unterschiedlichen cDNA Fragmente aus der Subtraktion auf Blatt-spezifische Gene wurden mittels Northern-Blott Analyse gegen RNA aus Blatt, Pfahlwurzel, Sproß und Infloreszenz hybridisiert. Dabei zeigten drei Klone, 2-1-36, 2-1-48 und 2-3-9 ein sehr spezifisches Hybridisierungsmuster. 2-1-36 und 2-1-48 hybridisierten nur mit RNA aus der Pfahlwurzel und nicht mit RNA aus Blatt, Sproß und Infloreszenz und 2-3-9 hybridisierte nur mit RNA aus grünem Gewebe und nicht mit RNA aus der Pfahlwurzel.

### Die Gene 2-1-36 und 2-1-48 werden während der Vegetationszeit ausschliesslich in der Zuckerrübenwurzel exprimiert.

Um das Expressionsverhalten der Gene 2-1-36 und 2-1-48 während der gesamten Vegetationszeit zu analysieren wird Zuckerrübensaatgut im Feld ausgelegt. Im Verlauf einer mitteleuropäischen Vegetationszeit werden 4, 6, 10, 12, 16 und 22 Wochen nach der Aussaat jeweils 5 vollständige Zuckerrübenpflanzen geerntet. Die Pflanzen zeigen zu keinem Zeitpunkt Krankheitserscheinungen. Gesamtzell-RNA wird nach Logemann et al., 1987 aus den Organen "sink" und "source" Blatt, Petiole Seitenwurzel und Pfahlwurzel (Wurzelkörper) isoliert. Die Expression der Gene wird durch RNA Blot Analysen bestimmt.

Für die Untersuchung der entwicklungsabhängigen Genexpression durch einen RNA-Blot werden jeweils 10 µg Gesamtzell-RNA pro Organ und Zeitpunkt in einem denaturierenden Formaldehyd-Agarosegel, wie bei Sambrook et al. (1989) beschrieben, aufgetrennt. Die elektrophoretisch aufgetrennte RNA wird durch Kapillar-Blot Technik (Sambrook et al., 1989) auf eine Hybond N Nylonmembran (Amersham Pharmacia Biotech, Freiburg) übertragen. Die radioaktive Markierung von 20 ng des 2-1-36 bzw. 2-1-48 cDNA-Fragmentes mit 50 µCi ³²P-dATP (6000 Ci/mMol, Amersham Pharmacia Biotech, Freiburg) erfolgt mit Hilfe des Prime-It 11 Random Primer Kit (Stratagene GmbH, Heidelberg) entsprechend der Herstellerangaben. Die anschließende Hybridisierung des RNA-Filters mit der markierten Sonde geschieht in 20 ml Hybridisierungspuffer (50% Formamid, 5 x SSC, 5 x Dendardts, 1% SDS, 0,1 mg Heringssperma-DNA, 40 mM Natriumphosphatpuffer pH 6,8) bei 42°C in einem Hybridisierungsofen (Biometra GmbH, Göttingen) nach Sambrook et al. 1989. Nach der Hybridisierung wird die Nylonmembran gewaschen und auf einem Röntgenfilm (Kodak BioMax MS, Kodak AG, Stuttgart) in Gegenwart einer Verstärkerfolie (Kodak BioMax MS Intensifying Screen, Kodak AG, Stuttgart) 6-24 h bei -80°C exponiert. Die Entwicklung des Röntgenfilm geschieht in Röntgenfilm-Entwickler und Röntgenfilm-Fixierer (Tetenal Photowerk GmbH und Co., Norderstedt).

Der mit der Sonde 2-1-48 hybridisierte RNA-Blot zeigt, daß das Gen 2-1-48 in 4 Wochen alten Zuckerrüben nur in der Wurzel und dem Wurzelkopf (Hypokotyl) und in 6, 10, 12, 16 und 22 Wochen alten Zuckerrüben ausschliesslich in der Pfahlwurzel und den Seitenwurzeln exprimiert wird (Abb. 1). Eine Expression des Gens 2-1-48 kann für die oberirdischen Pflanzenorgane Petiole, "source" und "sink" Blatt zu keinem Zeitpunkt beobachtet werden.

Der RNA-Blot wurde mit einem Phosphoimager (Bioimaging Analyzer BAS 1000, Fujiy Japan) ausgewertet, um die Transkriptakkumulation zu quantifizieren. Die Daten der Quantifizierung sind in Tabelle 1 wiedergegeben.

Die Akkumulation eines durch die Sonde 2-1-48 nachweisbaren Transkriptes und damit eine entsprechende Promotoraktivität ist in den Pfahlwurzeln von 4, 6 und 10 Wochen alten Pflanzen stark ausgeprägt. In 12 Wochen alten Wurzeln errreicht die Genexpression ihr Maximum und nimmt dann in 16 und 22 Wochen alten Pflanzen deutlich ab. Das Gen 2-1-48 wird somit in den Wurzelkörpern junger Rüben und Pflanzen mittleren Alters stark bzw. sehr stark exprimiert und nimmt dann mit zunehmendem Alter der Pflanzen deutlich ab. In den oberirdischen Planzenorganen kann nur bei jungen Rüben eine sehr geringe Genexpression in Petiolen und Blättern nachgewiesen werden. Die in 6, 10 und 12 Wochen alten Petiolen meßbare Transkriptmenge beträgt nur 3, 2 bzw. 1.2 % der zu diesen Zeitpunkten in der Pflahlwurzel meßbaren Transkriptmenge. Die in 6 und 10 Wochen alten "sink" und "source" Blättern gemessene Transkriptmenge beträgt 2.5% bzw. 1.6 und 2.1 % der zu diesen Zeitpunkten in der Pflahlwurzel akkumulierten 2-1-48 Transkriptes.

Die Verwendung des cDNA Klones 2-1-36 als Hybridisierungssonde für den entwicklungs-spezifischen RNA-Blot zeigt, daß das Gen in 4 Wochen alten Zuckerrüben nur in der Wurzel und dem Wurzelkopf (Hypokotyl) und in 6, 10, 12, 16 und 22 Wochen alten Pflanzen ausschließlich in der Pfahlwurzel und in den Seitenwurzeln exprimiert wird (Abb. 2). In den oberirdischen Organen Petiole, "sink" und "source" Blatt ist zu keinem Zeitpunkt ein Transkript sichtbar. Die Expression des Gens 2-1-36 ist in der Wurzel und den Seitenwurzeln nicht konstitutiv sondern erfolgt entwicklungsabhängig. Diese auf dem Autoradiogramm des RNA-Blots bereits sichtbare Tendenz wird nach der Quantifizierung des markierten Filters durch den Posphoimager noch deutlicher (Tabelle 2). Während das Gen 2-1-36 in der Jugendentwicklung der Rübe in 4, 6 und 10 Wochen alten Pfahlwurzeln bzw. 6 und 10 Wochen alten Seitenwurzeln schwach aber zunehmend stärker exprimiert wird, kommt es in Rüben und Seitenwurzeln nach 12 Wochen zu einem sehr starken Anstieg der Expression. Die Expression des Gens 2-1-36 fällt in den Pflahlwurzeln zu den Zeitpunkten 16. und 22. Woche im Vergleich zu dem 12-Wochenwert etwas ab, bleibt jedoch auf hohem Niveau. In den Seitenwurzeln 16 Wochen alter Pflanzen kommt es im Vergleich zum 12-Wochenwert ebenfalls zu einer Abnahme der 2-1-36 Expresion. Allerdings steigt die Expression von 2-1-36 in diesem Organ in den 22 Wochen alten Pflanzen wieder auf das hohe 12-Wochen-Niveau. Das Gen 2-1-36 wird somit in 4-10 Wochen alten Pflanzen schwach und in den 12-22 Wochen alten Pflanzen stark und damit entwicklungsabhängig exprimiert. In den oberirdischen Organen "sink"- und "source" Blatt sowie Petiole kann zu keinem Zeitpunkt eine signifikante Transkriptmenge quantifiziert werden. Sämtliche Meßwerte liegen bei diesen Organen in der Größenordnung des Hintergrundes.

### Das Gen 2-3-9 wird während der Rübenentwicklung ausschließlich in den oberirdischen Pflanzenorganen exprimiert.

Um die Expression des Gens 2-3-9 während der gesamten Vegetationszeit zu analysieren, wurden die für die Expressionsanalyse der wurzelspezifisch exprimierten Gene verwendeten RNA-Blots mit der Sonde 2-3-9 hybridisiert. Diese RNA-Blots waren unter Verwendung von RNA aus den Organen "sink"- und "source"-Blatt, Petiole, Pfahlwurzel und Seitenwurzel von 4, 6, 10, 12, 16 und 22 Wochen alten Zuckerrüben wie beschrieben angefertigt worden.

Das Hybridisierungsergebnis des RNA-Blots zeigt, wie in Abb. 3 dargestellt, daß das Gen 2-3-9 zu jedem Untersuchungszeitpunkt in "sink"- und "source"-Blättern sowie den Petiolen exprimiert wird. Eine Expression in den unterirdischen Pflanzenorganen Seiten- und Pfahlwurzel ist zu keinem Zeitpunkt optisch sichtbar. Die Quantifizierung des RNA-Blots mit Hilfe des Phospoimager bestätigt den optischen Eindruck und zeigt die Abwesenkeit von 2-3-9 Transkripten in der Pflahl- und den Seitenwurzeln und eine starke Transkriptakkumulation in Blättern sowie Petiolen (Tabelle 3). Die Meßwerte für die Pfahl- und Seitenwurzel sind rechnerisch Null bzw. liegen im Schwankungsbereich des Hintergrundes. Das Gen 2-3-9 wird somit während der gesamten Vegetationszeit ausschliesslich in den oberirdischen Pflanzenteilen exprimiert.

### Das Gen 2-3-9 liegt in unterschiedlicher Kopienzahl in verschiedenen Zuckerrüben Genotypen vor.

Die Zahl der genomischen Kopien des Gens 2-3-9 wird für die zwei Zuckerrübengenotypen 1 K0088 und 4B5421 bestimmt. Genomische DNA wird aus den Blättern der zwei Genotypen nach Saghai-Maroof et al (1984) isoliert. Jeweils 10 µg genomische DNA werden einzeln mit den Restriktionsenzymen *Eco*RI, *Hind*III, *Pst*I, *Sal*I*, Bam*HI, *Eco*RV *Xho*I und *Bgl*II geschnitten und die entstehenden DNA Fragmente in einem 0.8 %igen Agarosegel aufgetrennt. Die DNA Fragmente werden durch alkalischen Transfer auf eine Hybond N Nylonmembran (Amersham Pharmacia Biotech, Freiburg) übertragen. Die radioaktive Markierung von 20 ng des cDNA-Fragmentes 2-3-9 mit 50 µCi ³²P-dATP (6000 Ci/mMol, Amersham Pharmacia Biotech, Freiburg) sowie die Hybridisierung erfolgt genau wie für die RNA Blots beschrieben. Nach der Hybridisierung wird die Nylonmembran auf einem Röntgenfilm (Kodak BioMax MS, Kodak AG, Stuttgart) in Gegenwart einer Verstärkerfolie (Kodak BioMax MS Intensifying Screen, Kodak AG, Stuttgart) 16 h bei -80°C exponiert und der Röntgenfilm anschließend entwickelt.

Das Autoradiogramm des DNA-Blots zeigt, daß in dem Genom des Genotypen 1 K0088 zwei Kopien des Gens 2-3-9 und in dem Genotyp 4B5421 nur eine Genkopie vorliegt (Abb. 4). Diese Einschätzung resultiert aus der Beobachtung, daß der Restriktionsverdau der 1 K0088 DNA mit *Eco*RI und *Hind*III zu drei bzw. mit *Pstl* zu zwei Hybrisierungssignalen führt während die DNA von 4B5421 unter diesen Bedingungen mit *Eco*RI und *Pstl* ein und mit *Hind*III zwei Signale gibt.

### Isolierung eines vollständigen 2-1-48 cDNA Klons durch RACE

Bevor genomische DNA Fragmente mit dem Promotorbereich des Gens 2-1-48 identifiziert wurden, wurde zuerst eine potentiell vollständige 2-1-48 cDNA rekonstruiert. Dafür wurde der "Marathon cDNA Amplification Kit" von CLONTECH eingesetzt. Marathon cDNA Amplifikation ist eine Methode, um 5' und 3' RACE (rapid amplification of cDNA ends) vom gleichen Template durchzuführen. Es wurden insgesamt sechs RACE Produkte für den 5'-Bereich und vier RACE Produkte für den 3'-Bereich sequenziert. Durch Vergleich der Sequenzen untereinander und mit dem ursprünglichen cDNA Fragment 2-1-48 konnte die Sequenz eines potentiell vollständigen cDNA Fragmentes rekonstruiert werden. Die aus den RACE Produkten rekonstruierte cDNA ist insgesamt 841 Basenpaare lang und entspricht in etwa der Größe des in Northern Blot Hybridisierungen nachgewiesenen pfahlwurzelspezifischen Transkriptes (ca. 800 Bp). Die Translation aller drei möglichen Leseraster ergab einen langen ununterbrochenen Leseraster von 150 Aminosäuren. Alle anderen möglichen Translationsprodukte enthielten zahlreiche Stopkodons. Das 150 Aminosäuren lange Protein zeigt eine Homologie mit dem 152 Aminosäure langen Major Latex Protein Homolog aus *Mesembryanthemum crystallinum.* 66 % der Aminosäuren beider Proteine (99 von 149) sind identisch. Die Funktion des Proteins ist nicht bekannt. Die 5'-Enden der cDNA Klone der Gene 2-1-36 und 2-3-9 wurden wie für das Gen 2-1-48 beschrieben durch 5'RACE amplifiziert und isoliert.

### Isolierung der Promotoren der Gene 2-1-48 und 2-1-36

Die Promotorbereiche in genomischen Klonen der Gene 2-1-48 und 2-1-36 wurden identifiziert und subkloniert. Dazu wurden aus einer genomischen Bank des Zuckerrüben Genotyps 1 K0088 Klone isoliert, die homologe Sequenzen zu den cDNA Klonen 2-1-48 und 2-1-36 tragen. Die Bank ist im Lambda Vektor EMBL3 SP6/T7 angelegt worden und enthält genomische Fragmente mit einer Durchschnittsgröße von 20 Kb. Für die Klonierung wurde die genomische DNA partiell mit Mbol gespalten und in die BamH1 Schnittstelle von EMBL3 ligiert. Die Inserts können mit Xhol herausgeschnitten werden. Für die Isolierung genomischer Klone wurden in einem Plaque-Hybridisierungsexperiment ca. 300 000 genomische Klone mit dem cDNA Fragmenten 2-1-48 und 2-1-36 hybridisiert (Sambrook et al., 1989). Hybridisierende Phagenplaques wurden gestochen und so lange replattiert bis nur noch der jeweilige hybridisierende Phagenklon im Ansatz vorhanden war. Es konnten insgesamt dreizehn Klone mit dem cDNA Fragment 2-1-48 und 10 Phagenklone mit dem cDNA Fragment 2-1-36 aufgereinigt werden.

In diesen Klonen wurde nun der genomische Bereich 5' zur rekonstruierten vollständigen cDNA identifiziert und subkloniert. Dazu wurde in der cDNA eine Restriktionsschnittstelle für ein Restriktionsenzym gesucht, welches relativ nah am 5'-Ende der cDNA liegt. Für Klon 2-1-48 wurde eine Ncol Schnittstelle identifiziert, die ca. 175 Basenpaare vom Transkriptionsstart entfernt ist. Die genomischen Klone, die mit dem cDNA Fragment 2-1-48 isoliert wurden, wurden mit Xhol und Ncol gespalten, gelelektrophoretisch aufgetrennt und mit einem radioaktiven Oligonukleotid hybridisiert, das 5' zur Ncol Schnittstelle liegt. Diese Hybridisierung identifizierte das Ncol/Xhol Fragment, welches genomische Sequenzen 5' zur rekonstruierten cDNA enthielt. Diese Fragmente wurden aus drei verschiedenen Lambdaklonen (L1, L12, L9) nach Auffüllen der Enden mit Klenow-Polymerase in einen Smal geschnittenen Plasmid-Vektor (pBluescript 11 SK) subkloniert. Bakterielle Kolonien mit rekombinanten Plasmiden wurden mittels Koloniehybridisierung identifiziert. Es wurden pro Lambdaklon drei (L12-14, L12-15, L12-16; L-9-10, L-9-11, L-9-12) bzw zwei (L1-05, L1-06) rekombinante Plasmide von beiden Seiten ansequenziert. An den Enden aller Klone konnte jeweils die Ncol Schnittstelle aus der cDNA und die Xhol Schnittstelle aus dem Polylinker des Lambdavektors identifiziert werden. Die subklonierten Xhol/Ncol Inserts der drei Lambdaklone sind jeweils ca. 7000 (L1), 4000 (L9) und 5000 (L12) Basenpaare lang. Das 4089 Basenpaare lange Insert des für die Subklonierung verwendeten XhoI/NcoI Fragmentes ist als SEQ ID NO: 1 dargestellt. Die Nukleotide 1-3967 umfassen den gesamten regulatorischen 5'-Bereich des Gens und damit den Promotor 2-1-48. Die transkribierte, nichttranslatierte DNA Sequenz erstreckt sich nach dem Vergleich mit dem 5'-Ende eines vollständigen cDNA Klons von Position 3911-3967. Die Positionen 3968-4089 entsprechen dem 5'-Ende des codierenden Bereichs. Die in dem Plasmid L9 vorliegende DNA-Sequenz unterscheidet sich aufgrund der Klonierunstechnik von der DNA-Sequenz der SEQ ID NO: 1 dadurch, daß das Basenpaar an Position 1 und 4089 fehlt.

Aus der isolierten DNA der Phagen, die mit dem cDNA Klon 2-1-36 hybridisieren, wurde unter Verwendung eines für den 5'-Bereich des cDNA Klons 2-1-36 spezifischen Oligonukleotids ein 1.923 kb großes Ndel-Ndel Fragment, das den Promotor des Gens 2-1-36 enthält, identifiziert. Die DNA-Enden des Ndel-Ndel Fragmentes wurden durch Klenow-Behandlung geglättet und der Promotor in den mit dem Restriktionsenzym Smal geschnittenen Vektor pBluescript II SK kloniert. Die Nukleotidsequenz des subklonierten Fragmentes wurde bestimmt. Von der analysierten Sequenz sind 1919 bp als SEQ ID NO: 2 dargestellt. Die Nukleotide 1-1840 umfassen den gesamten subklonierten regulatorischen 5'-Bereich des Gens und damit den Promotor 2-1-36. Die transkribierte, nichttranslatierte DNA Sequenz erstreckt sich nach dem Vergleich mit dem 5'-Ende eines vollständigen cDNA Klons von Position 1606-1840. Die Positionen 1841-1919 stellen die ersten 79 translatierten Basenpaare des Gens dar.

### Isolierung der Promotoren der beiden 2-3-9-Gene

Die Promotorbereiche der in zwei Kopien in dem Zuckerrüben Genotyp 1 K0088 vorliegenden Gene 2-3-9 wurden identifiziert und isoliert. Dazu wurden aus der genomischen Bank des Genotyps 1 K0088 Lambda-Phagen isoliert, deren Inserts eine Homologie zu dem cDNA Klon 2-3-9 aufweisen. Insgesamt 300 000 Phagen der im Vektor EMBL 3 SP6/T7 angelegten Bank wurden nach dem "Lambda Library Protocol Handbook", Clontech, PT 1010-1) unter Verwendung des E. coli Stammes K802 in geschmolzener LB Top Agarose + 10 mM MgSO₄ auf 150 mm großen Petrischalen, die das Medium LB + 10 mM MgS0₄ enthalten, ausgebracht. Die Konzentration an Phagen betrug 25 000 pro Platte. Um die ausgebrachten Phagen nach den Promotoren der Gene 2-3-9 zu durchsuchen wurden jeweils 2 Replikate von Nylonfiltern (NEN Life Science Products, #NEF 978Y) pro Platte angefertigt. Die Filter wurden für ein Hybridisierungsexperiment mit dem radioaktiv markierten cDNA Fragment 2-3-9 hybridisiert. Zwölf positiv hybridisierende Phagen wurden mit Hilfe einer Parteurpipette aus den Platten ausgestanzt, durch entsprechende mikrobiologische Verdünngsschritte vereinzelt und bis zur Reinheit aufgereinigt. Die Reinheit der Phagenisolate wurde nach jedem Arbeitsschritt durch eine radioaktive Hybridisierung überprüft. Aus 9 positiven Lambda-Phagen wurde DNA unter Verwendung des Qiagen Lambda-DNA Präparationskits (Qiagen, Hilden, Deutschland) isoliert. Durch eine Kombination von Long Distance-PCR (LD-PCR) und Restriktionsanalysen wurde die Lage und die Orientierung des codierenden Bereichs des Gens 2-3-9 und damit des Promotors mit Bezug auf den linken und rechten Phagenarm bestimmt.

Für die Amplifikation der klonierten genomischen Fragmente durch LD-PCR wurde eine Primerkombination eingesetzt bei der ein handelsüblicher 5'- und 3'-Primer im linken bzw. rechten Phagenarm ausserhalb der klonierten Zuckerrüben DNA bindet. Die für die Phagenarme spezifischen 5'- und 3'-Primer des "EMBL3 LD-Insert Screening Amplimer Set" (Clontech #9104-1, Heidelberg, Deutschland) haben die Nukleotidsequenz CTG CTT CTA ATA GAG TCT TGC TGC AGA CAA ACT GCG CAA C (SEQ ID NO: 5) bzw. TGA ACA CTC GTC CGA GAA TAA CGA GTG GAT CTG GGT C (SEQ ID NO: 6). Die Amplifikation der genomischen Zuckerrüben DNA Fragmente erfolgte mit Hilfe des "Advantage Genomic PCR Kit" (Clontech # K1906-1, Heidelberg, Deutschland).

Die PCR Bedingungen lauteten bei Verwendung von 100 ng Lambda-DNA, einer Primerkonzentration von 0.4 µM, 0.5 µl Tth Polymerase-Mix und 25 µl Reaktionsvolumen in einem Multicycler PTC-200 (MJ Research, Watertown, MA., USA) folgendermaßen:

| | | |
|---|---|---|
| 1 x | Schritt 1: 1 min | 95°C |
| 25 x | Schritt 2: 15 sec | 95°C |
| | Schritt 3: 24 min | 68°C |
| 1 x | Schritt 5: 10 min | 68°C |

Weiterhin wurden für analytische Zwecke die 5'- und 3'-Primer des "EMBL3 LD-Insert Screening Amplimer Set" jeweils in Kombination mit Primern S82 und S83 eingesetzt, die für den 5'- und 3'-Abschnitt des codiernden Bereichs des Gens 2-3-9 spezifisch sind. Dadurch konnte die Größe der sich an den Leserahmen anschließenden genomischen Fragmente bestimmt werden. Die Primier S82 und S83 haben die Sequenz AGG TTA TCA AAA GGC CCC TTT CCA GTC A (SEQ ID NO: 7) und GTT TGT GCA AGC CGA GCT GGT GAA CGC C (SEQ ID NO: 8). Die PCR Bedingungen entsprechen bei einer auf 20 ng reduzierten DNA Menge den zuvor beschriebenen LD-PCR Bedingungen.

Für die Restriktionsanalysen der Lambda-Klone wurden jeweils 200-400 ng isolierte Phagen-DNA oder 200 ng LD-PCR Produkte einzeln mit den Restriktionsenzymen *Cla*I, *Eco*RI, *Eco*RV, *Hind*III, *Pst*I*, Sac*I, *Sal*I, *Xho*I und der Enzymkombination von *Pst*I/*Sa*lI und *Cla*I/*Sal*I geschnitten. Die DNA-Fragmente wurden in einem 0.8 % Agarosegel aufgetrennt, auf Nylonmembranen transferiert und mit dem radioaktiv markierten cDNA Klon 2-3-9 nach Sambrook et al., 1989 hybridisiert. Die Auswertung der PCR- und Restriktionsanalysen ergab, daß 8 Lambda-Klone die genomische Kopie 1 des Gens 2-3-9 enthalten und nur ein Klon die Kopie 2 des Gens 2-3-9 enthält.

### Subklonierung und Charakterisierung des in oberirdischen Organen aktiven C1-Promotors

Ausgehend von den 8 Lambda-Klonen, die die genomische Kopie 1 des Gens 2-3-9 tragen, wurde der Phage λ c6.1.1 für die Subklonierung des Promotors ausgewählt. Der Promotor der Kopie 1 des Gens 2-3-9 wird im nachfolgenden als C1 Promotor bezeichnet. Der vollständige codierende Bereich des Gens sowie der C1 Promotor und der regulatorische 3'-Bereich des Gens sind auf einem 6.294 kb großen *Cla*I-*Cla*I Fragment lokalisiert. Das *Cla*I-*Cla*I Fragment wurde aus der DNA des Phagen λ c6.1.1 isoliert und in den mit *Cla*I geschnittenen und nach alkalischer Phosphatase Behandlung dephosphorylierten Vektor pBluescript II KS+ (Stratagene, Heidelberg, Deutschland) subkloniert. Das resultierende Plasmid trägt die Bezeichnung pc1a. Die Nukleotidsequenz des subklonierten Fragmentes mit dem genomischen Fragment der Kopie 1 des Gens 2-3-9 wurde bestimmt. Von der bestimmten Nukleotidsequenz sind 1148 bp als SEQ ID NO: 3 dargestellt. Die Nukleotide 1-1097 umfassen den gesamten auf dem Plasmid pc1a lokalisierten regulatorischen 5'-Bereich des Gens und somit den C1-Promotor. Die transkribierte, nicht-translatierte DNA Sequenz erstreckt sich nach dem Vergleich mit dem 5'-Ende der entsprechenden cDNA von Position 984-1097. Die Position 1098-1148 stellt die ersten 51 translatierbaren Basenpaare des Gens dar. Der sich an den C1 Promotor anschließende genomische 5'-Bereich wurde ebenfalls aus dem Phagen λ c6.1.1 subkloniert. Dazu wurde die Phagen-DNA mit den Restriktionsenzymen *Sal*I und *Cla*I geschnitten, das isolierte 6.026 kb große *Sal*I-*Cla*I Fragment in die korrespondierenden Schnittstellen des Vektors pBluescript II KS+ subkloniert. Das resultierende Plasmid trägt die Bezeichnung pc1 b (Abb. 5).

### Fusion des Zuckerrüben C1 Promotors mit dem Luciferasegen aus Photinus pyralis

Um die Aktivität des isolierten C1 Promotors in Zuckerrübenblättern nachzuweisen, wurde der C1 Promotor mit dem Luciferasegen aus *Photinus pyralis* translationell fusioniert. Der Reportergenvektor pGEM-luc (Promega, Mannhein, Deutschland), der das *P. pyralis* Luciferasegen trägt, wurde mit dem regulatorischen 3'-Bereich des Nopalinsynthase-(*nos*)-Gens versehen, um einen für die Expression in Pflanzen geeigneten Vektor zu erhalten. Dazu wurde der Vektor pBI101.3 (Clontech, Heidelberg, Deutschland) mit *Eco*RI linearisiert und die *Eco*RI DNA Enden durch Klenow Behandlung in stumpfe DNA Enden überführt. Durch Nachschneiden mit *Sac*I konnte der 0.26 kb große *nos*-Terminator freigesetzt und anschließend isoliert werden. Der Vektor pGEM-luc wurde mit der Restriktionsendonuklease *Sfi*I linearisiert und die Schnittstelle durch T4-Polymerase Behandlung geglättet. Nach Restriktion des so vorbehandelten Vektors mit dem Restriktionsenzym *Sac*I wurde der isolierte 0.26 kb großen nos-Terminators als *Eco*RI (aufgefüllt)-*Sac*I Fragment in pGEM-luc eingefügt. Der resultiernde Vektor trägt die Bezeichnung pluc-nos2 (Abb. 6). Das C1 Promotorfragment wurde von Position 1-1145 (SEQ ID NO: 3) durch Nutzung der Restriktionsenzyme *Sal*I und *Avi*II aus dem Plasmid pc1 a herausgeschnitten, die DNA Enden durch Klenowbehandlung geglättet und das Promotorfragment isoliert. Der Vektor pluc-nos2 wurde mit dem Restriktionsenzym *Apa*I linearisiert und die DNA Enden durch T4 Polymerase Behandlung geglättet. Nach Dephosphorylierung des Vektors wurde der C1 Promotor als *Sal*I (aufgefüllt)-AviII Fragment subkloniert. Der resultierende Vektor trägt die Bezeichnung pc1 L-1097 (Abb. 7). In dem Vektor pc1L-1097 ist der Promotor über die für die ersten 16 Aminosäuren des Gens 2-3-9 codierenden Basenpaare translationell mit dem Luciferasegen verbunden. Um die Frage zu beantworten, ob das in pc1L-1097 verwendete C1 Promotorfragment für die Promotoraktivität ausreichend ist, wurde dieses regulatorische DNA-Fragment um den sich in der Zuckerrübe daran anschließenden genomischen 5'-Bereich erweitert. Dazu wurde das 6029 bp große DNA-Fragment aus pc1b verwendet. Das Plasmid pc1b wurde mit dem Restriktionsenzym *Kpn*I geschnitten und die DNA-Enden durch T4-Polymerase-Behandlung geglättet. Durch Nachschneiden mit dem Restriktionsenzym *Not*I konnte der genomische Bereich als *Kpn*I (geglättet)-*Not*I Fragment isoliert und in den Vektor pc1 L-1 097 kloniert werden. Der Vektor pc1 L-1 097 war zuvor mit *Hind*III linearisiert, die DNA-Enden mit dem Klenow-Fragment geglättet und dann nochmals mit dem Restriktionsenzym *Not*I behandelt worden. Das resultierende Plasmid trägt die Bezeichnung pc1L-7126 (Abb. 8) und umfaßt den 5'-Bereich der Kopie 1 des Gens 2-3-9 von Position 1-7126.

### Nachweis der C1 Promotoraktivität in Zuckerrübenblättern durch ballistische Transformation

Die Aktivität des Reportergenkonstruktes pc1L-1097 und pc1L-7126 wurde in Blättern nach ballistischer Transformation gemessen. Die ballistische Transformation wurde unter Verwendung eines PDS-1000/He Particle Delivery Systems (BioRad) in Anlehnung an die Herstellerangaben durchgeführt. Als Microcarrier wurde Goldpulver Typ 200-03 (Heraeus, Hanau, Deutschland) mit einer Partikelgröße von 1,09-2,04 µm verwendet. Die Vorbereitung und das Beladen der Microcarrier mit den Reportergenkonstrukten erfolgte entsprechend dem BioRad Protokoll US/EG Bulletin 1688. Die Vektoren pc1 L-1 097 und pc1 L-7126 wurden in einem äquimolaren Verhältnis verwendet. Um Ergebnisschwankungen, die auf unterschiedliche Transformationseffizienzen und nicht auf Unterschiede der Promotorstärken zurückzuführen sind, auszuschließen, wurde eine Normaliserung der Genexpression durchgeführt. Dazu wurde das Plasmid p70Sruc mit dem Luciferasegen aus *Renilla reniformis* als zweites Reportergensystem in einem Volumenverhältnis von 7:3 mit den Vektoren pc1L-1097 bzw. pc1L-7126 gemischt und für die Beladung der Microcarrier verwendet. Die Messung einer weiteren Reportergenaktivität, deren Expression unter der Kontrolle des zweifachen 35S-Promotors steht, erlaubt es, dieses Ergebnis als Referenz für die Berechnung der Transformationseffizienz des einzelnen Experimentes zu verwenden.

Für jedes zu untersuchende Reportergenkonstrukt wurden drei Beschußexperimente durchgeführt, die Genexpression normalisert und dann der Mittelwert der normalisierten Genexpression errechnet. Zu Kontrollzwecken wurden zwei Beschußexperimente mit Goldpulver ohne DNA-Beladung durchgeführt. Die so ermittelten Enzymaktivitäten stellten die endogene Hintergundaktivität in den Blättern dar und wurden von den übrigen Versuchswerten abgezogen. Pro Beschußexperiment wurden 13 Blattrondelle mit einem Korkbohrer (Größe 8) aus jungen bzw. alten Zuckerrübenblättern ausgestanzt und 6h in 90 mm großen Petrischalen auf mit Agar verfestigten MS-Medium + 0.4 M Mannitol bei 25°C osmotisch vorbehandelt. Die Beschußparameter waren 1550 psi Berstscheibenstärke, 9 cm Abstand zwischen Blattrondellen und Berstscheibe und 27.5 in Hg Unterdruck in der Gerätekammer. Nach dem Beschuss wurden die Platten 16h bei 25°C unter Beleuchtung inkubiert.

Die *Photinus* und die *Renilla* Luciferaseaktivität wurden mit dem Dual-Luciferase Reporter Assay System (Promega, Mannheim, Deutschland) in einem Lumat 9501 Luminometer (PE Biosystem) entsprechend den Herstellerangaben bestimmt. Die Gewinnung eines für die Messung geeigneten Enyzmextraktes erfolgte durch Zermörsen der Blattrondelle eines Beschussexperimentes in flüssigem Stickstoff. Nach Verdampfen des Stickstoffes wurden das pulvrige Blattmaterial mit dem 10 fachen Volumen (v/w) an Passive Lysis Buffer (PLB) homogenisiert. Der flüssige Überstand wurde in ein 1.5 ml Eppendorfgefäß überführt und 5 min bei 4°C und 20 000 g abzentrifugiert. Der klare Überstand wurde abgenommen und jeweils 10 µl Rohextrakt für die *Photinus* und die *Renilla* Luciferaseaktivitätsmessung eingesetzt.

Der Mittelwert der normalisierten Genexpression betrug bei Verwendung des Konstruktes pc1 L-1 097 für kleine Blätter 8,0 und für große Blätter 9,6 (Tabelle 4). Für das Konstrukt pc1L-7126 betrug der Mittelwert der normalisierten Genexpression 3,2 bei kleinen Blättern und 7,0 bei großen Blättern (Tabelle 4). Damit ist das kürzere C1 Promotorfragment des Plasmides pc1 L-1 097 in Rübenblättern nicht nur ausreichend für die Expression des Reportergens sondern aktiver als das längere C1 Promotorfragment des Konstruktes pc1L-7126.

### Subklonierung und Charakterisierung des in oberirdischen Organen aktiven C2-Promotors

Die Subklonierung des Promotors der 2. Kopie des Gens 2-3-9 erfolgte aus dem Phagen λ c7.2.1. Der Promotor dieses Gens wird im folgenden als C2 Promotor bezeichnet. Die vorangegangenen Restriktionsanalysen hatten gezeigt, daß der 5'-Bereich des codierenden Bereichs sowie ca. 3.0 kb des regulatorischen 5'-Bereichs der 2 Kopie des Gens 2-3-9 auf einem 4.002 kb großen *Pst*I-*Pst*I Fragment des Phagen λ c7.2.1 gelegen sind.

Das 4.002 kb große *Pst*I*-Pst*I Fragment des Phagen λ c7.2.1 wurde nach einem *Pstl* Restriktionsverdau isoliert und in den Vektor pBluescript ll KS+ (Stratagene, Heidelberg, Deutschland) kloniert. Der Vektor pBluescript ll KS+ wurde dazu ebenfalls mit dem Restriktionsenzym *Pstl* geschnitten and anschließend durch Behandlung mit der alkalischen Shrimps Phosphatase (Roche Diagnostics GmbH, Mannheim, Deutschland) dephosphoryliert. Nach Ligation und Transformation in den *E. coli* Stamm XL-1 wurde Plasmid DNA aus den *E. coli* Transformanten isoliert und positive Klone wurden durch Restriktionsanalysen identifiziert. Das resultierende Plasmid trägt die Bezeichnung pc2. Dle Nukleotidsequenz des 4.002 kb großen *Pst*I-*Pst*I Fragmentes wurde bestimmt. Von der Nukleotidsequenz sind 3049 bp als SEQ lD NO: 4 dargestellt. Die Nukleotide 1-2998 umfasssen den gesamten auf dem Plasmid pc2 lokalisierten, regulatorischen 5'-Bereich des Gens und damit den C2 Promotor. Die transkribierte, nicht-translatierte DNA Sequenz erstreckt sich nach dem Vergleich mit dem 5'-Ende der entsprechenden cDNA von Position 2928-2998. Die Position 2999-3049 stellt die ersten 51 translatierten Basenpaare des Gens dar.

### Fusion des Zuckerrüben C2 Promotors mit dem Luciferasegen aus Photinus pyralis und Erstellung von Deletionskonstrukten

Um die Aktivität des isolierten C2 Promotors in Zuckerrübenblättern nachzuweisen, wurde der C2 Promotor mit dem Luciferasegen aus *Photinus pyralis* translationell fusioniert. Dazu wurde das in pc2 subklonierte 4002 bp große DNA Fragment, dass den C2 Promotor und den 5'-Bereich des codierenden Bereich der 2. Kopie des Gens 2-3-9 umfasst, nach einem Restriktionsverdau mit den Enzymen *Not*I und *Eco*RI isoliert. Das DNA Fragment wurde dann partiell mit dem Enzym *Avi*II geschnitten und ein ca. 3100 bp großes *Not*I-*Avi*II Fragment isoliert, das die Basenpaare 1-3046 der SEQ lD NO: 4 enthält. Das *Not*I-*Avi*II Fragment wurde dann in den Reportergenvektor pluc-nos2 subkloniert. Dazu wurde der Vektor pluc-nos2 zunächst mit dem Restriktionsenzym *Apa*I geschnitten und die überstehenden DNA Enden durch T4-Polymerase Behandlung geglättet. Durch nochmaliges Schneiden mit dem Restriktionsenzym *Not*I wurde der Vektor pluc-nos2 in eine für die Aufnahme des *Not*I-*Avi*II Fragmente geeignetes Zustand überführt. Das resultierende Plasmid trägt die Bezeichnung pc2L-2998 (Abb. 9). Der Vektor pc2L-2998 trägt die C2 Promotorsequenz der SEQ lD NO: 4 von Nukleotidposition 1-2998 sowie die ersten 48 translatierten Basenpaare des Gens 2-3-9 von Position 2999-3046.

Um die minimale DNA-Fragmentgröße zu identifizieren, die für die Aktivität des C2 Promotors notwendig ist, wurden drei 5'-Deletionskonstrukte erstellt. Die Konstrukte pc2L-1827 , pc2L-989 und pc2L-342 enthalten die C2 Promotorsequenz der SEQ ID NO: 4 von Nukleotidposition 1172-2998, 2011-2998 bzw. 2657-2998 sowie die ersten 48 translatierten Basenpaare des Gens 2-3-9 von Position 2999-3046 (Abb. 10-12). Die Vektoren wurden ausgehend von pc2L-2998 im einzelnen wie folgt entwickelt:
Vektor pc2L-1827 durch einen Restriktionsverdau mit den Enzymen *Kpn*I und *Not*I, einem nachfolgendem Glätten der DNA-Enden durch T4-Polymerasebehandlung und der Religation des Vektors.
Plasmid pc2L-989 durch einen Verdau mit dem Restriktionsenzym *Sma*I und der Religation des Vektors.
Plasmid pc2L-342 durch einen Verdau mit dem Restriktionsenzym *Not*I und einem partiellen *Sal*I Verdau. Nach dem Glätten der DNA-Enden durch eine Klenowbehandlung wurde der Vektor religiert.

### Nachweis der C2 Promotoraktivität in Zuckerrübenblättern durch ballistische Transformation

Die Aktivität der Reportergenkonstrukte pc2L-2998, pc2L-1827, pc2L-989 und pc2L-342 wurde in Blättern nach ballistischer Transformation gemessen. Die ballistische Transformation und die Bestimmung der Reportergenaktivitäten wurde wie zuvor für den C1 Promotor beschrieben durchgeführt. Die Vektoren pc2L-2998, pc2L-1827, pc2L-989 und pc2L-342 wurden in einem äquimolaren Verhältnis verwendet. Um Ergebnisschwankungen, die auf unterschiedliche Transformationseffizienzen und nicht auf Unterschiede der Promotorstärken zurückzuführen sind, auszuschließen, wurde eine Normaliserung der Genexpression unter Verwendung des Plasmides p70Sruc durchgeführt. Das Plasmid p70Sruc trägt das Luciferasegen aus *Renilla reniformis* als zweites Reportergensystem. Für jedes zu untersuchende Reportergenkonstrukt wurden vier Beschussexperimente pro DNA-Präparation durchgeführt. Für die Konstrukte pc2L-2998 und pc2L-1827 wurde eine und für Konstrukte pc2L-989 und pc2L-342 wurden zwei DNA-Präparationen verwendet. Die gemessene Genexpression wurde normalisert und dann der Mittelwert der normalisierten Genexpression errechnet. Zu Kontrollzwecken wurden vier Beschußexperimente mit Goldpulver ohne DNA-Beladung durchgeführt.
Der Mittelwert der normalisierten Genexpression betrug bei Verwendung der Konstrukte pc2L-2998, pc2L-1827, pc2L-989 und pc2L-342 im einzelnen 8.0, 4.5, 6.45 und 6,45 (Abb. 13). Damit sind alle C2 Promotorfragmente geeignet, um in Blättern ein Gen zur Expression zu bringen. Das kleinste Promotorfragment des Konstruktes pc2L-342, das der Nukleotidsequenz der SEQ ID NO: 4 von Position 2657-3046 entspricht, ist genauso aktiv wie die größeren Promotorfragmente der Konstrukte pc2L-2998, pc2L-1827 und pc2L-989.

### Konstruktion von Pflanzentransformationsvektoren am Beispiel von pc1 G-1097 und pc2G-2998

Für die stabile Transformation der Genfusionen aus den in oberirdischen Organen aktiven C1 und C2 Promotoren und dem *gus*-Reportergen werden die binären Vektoren pc1 G-1097 (Abb. 14) und pc2G-2998 (Abb. 15) entwickelt.

Für die Konstruktion von pc1 G-1097 wird der C1 Promotor aus dem Vektor pc1L-1097 als ca. 1.17 kb großes *Hind*III-*Bam*HI Fragment isoliert und in den mit *Hind*III und *Bam*HI linearisierten binären Vektor pB1101.3 (Clontech, Heidelberg) eingefügt. Der umklonierte C1 Promotor umfaßt die Nukleotidsequenz der SEQ ID NO: 3 von Position 1-1145. In dem resultierenden Vektor pc1 G-1097 ist der C1 Promotor über die für die ersten 16 Aminosäuren des Gens 2-3-9 codierenden Basenpaare translationell mit dem *gus*-Gen aus pBI1.101.3 verknüpft.

Der Vektor pc2G-2998 wird in der Weise konstruiert, daß der C2 Promotor aus dem Vektor pc2L-2998 isoliert und translationell mit dem *gus*-Gen aus pBl101.3 verknüpft wird. Der Vektor pc2G-2998 trägt wie das Plasmid pc2L-2998 die C2 Promotorsequenz der SEQ ID NO: 4 von Nukleotidposition 1-2998 sowie die ersten 48 translatierten Basenpaare des Gens 2-3-9 von Position 2999-3046. Dazu wurde pc2L-2998 mit dem Restriktionsenzym *Pst*I geschnitten und die DNA-Enden wurden durch Klenow-Behandlung geglättet. Nach der Verwendung des Restriktionsenzyms *Bam*HI konnte der C2-Promotor als ca. 3070 bp großes DNA-Fragment isoliert in den entsprechend präparierten binären Vektor pB1101.3 kloniert werden. Für die Aufnahme des C2 Promotors wurde der Vektor pBI101.3 zunächst mit dem Restriktionsenzym *Sal*I linearisiert und die DNA-Enden durch Klenow-Behandlung aufgefüllt. Anschließend wurde der Vektor mit dem Enzym *Bam*HI geschnitten.

### Transformation der Konstrukte pc1G-1097 und pc2G-2998 in Pflanzen

Die für die Produktion transgener Pflanzen vorgesehenen Konstrukte werden zunächst durch ein direktes DNA-Transformationsverfahren (An, 1987) in den *Agrobacterium tumefaciens* Stamm GV2260 überführt. Die Selektion rekombinater *A. tumefaciens* Klone erfolgt unter Verwendung des Antibiotikums Kanamycin (50 mg/l). Nachfolgend ist die Transformation beispielhaft für den Vektor pc1G-1097 beschrieben.

Die Reportergenkassette bestehend aus der translationellen Fusion zwischen dem C1-Promotor und dem gus-Gen wird mit Hilfe von *A. tumefaciens* nach Horsch et al. (1985) in den Sommerraps Genotyp Drakkar transformiert. Transgene Pflanzen werden unter Verwendung des Antibiotikums Kanamycin selektioniert. Die Anwesenheit des Promoters in den transgenen Pflanzen kann durch PCR überprüft werden. Die Verwendung der Primer GTGGAGAGGCTATTCGGTA (SEQ ID NO: 9) und CCACCATGATATTCGGCAAG (SEQ ID NO: 10) führt zu der Amplifikation eines 553 bp großen DNA-Fragments aus dem *npt*II Gen. Die PCR wird unter Verwendung von 10 ng genomischer DNA, einer Primerkonzentration von 0,2 µM bei einer Annealingtemperatur von 55°C in einen Multicycler PTC-200 (MJ Research, Watertown, USA) durchgeführt.

Unter Anwendung der beschriebenen Techniken wurden mit dem binären Vektor pc1 G-1097 jeweils zwanzig unabhängige Raps- und Tabaktransformanten und mit dem binären Vektor pc2G-2998 zwanzig unabhängige Raps- und Kartoffeltrans-formanten gewonnen.

### Nachweis der C1 und C2 Promotoraktivität in Blättern transgener Raps-, Kartoffel- und Tabakpflanzen

Um die Aktivität der C1 und C2 Promotoren in den Blättern transgener Raps- und Tabakpflanzen nachzuweisen wird eine histochemische GUS-Färbung durchgeführt. Blätter werden von transgenen und nichttransgenen *in vitro* Pflanzen abgenommen, mit GUS-Färbelösung (2 mM 5-Bromo-4-chloro-3-indoyl-beta-glucuronid, 50 mM Natrium-phosphat pH 7,0, 0,5% Triton X-100, 2 % N, N,-Dimethylformamid) für 15 sec vakuuminfiltriert und anschließend für 16h bei 37°C inkubiert. Anschließend wird das Chlorophyll der Blätter mit 70%igen Ethanol extrahiert. Die Blaufärbung des Gewebes zeigt die Bereiche an, in denen GUS-Aktivität vorliegt und der Promotor exprimiert wird.

Blattstückchen der Raps-Transformante pc1G-1097-86 sowie der Tabaktransformante pc1G-1097-3 zeigen eine intensive gleichmäßige Blaufärbung des Gewebes (Abb. 16 und 17). Im Vergleich dazu sind die Blattstückchen der nicht-transgenenRaps- bzw. Tabakpflanzen nach dieser Behandlung weiß. Ebenfalls intensiv blaugefärbt werden die Blattstückchen der Kartoffeltransformante pc2G-2993-1. Diese Ergebnisse zeigen, daß die C1 und C2 Promotoren in stabil transformierten Raps-, Kartoffel- und Tabakpflanzen funktionell sind und daß das gus-Reportergen unter der Kontrolle des Promotors in Familien der Brassicaceae und Solanaceae exprimiert wird.

### Homologie zwischen den C1 und C2 Promotoren

Der Nukleotidsequenzvergleich zwischen den DNA Sequenzen der Promotoren C1 und C2 zeigt, daß der Promotorbereich des C1 Promotors von Position 780-1051 mit der Ausnahme von 3 Basenpaaren vollkommen identisch mit der Sequenz des C2 Promotors von Position 2707-2984 ist (Abb. 19). Die Sequenzen des C1 Promotors von Position 1-799 zeigen keine signifikante Homologie zu der DNA-Sequenz des C2 Promotors von Position 1-2706. Der homologe Bereich zwischen den Promotoren umfaßt mit Bezug auf den Translationsstart die Positionen von -320 bis -42 des C1 Promotors und die Positionen von -292 bis -21 des C2 Promotors. In dem homologen Promotorbereich liegen neben der TATA Box und dem jeweiligen Transkriptionsstart mehrere zwischen beiden Promotoren konservierte cis-Elemente. Die TATA-Box erstreckt sich in dem C1 Promotor von Position 950-956 und in dem C2 Promotor von Position 2877-2883. Neben der TATA Box findet sich von den in Promotoren häufig gefundenen Elementen eine CAAT Box bei Position 884-887 im C1-Promotor und bei Position 2811-2814 im C2-Promotor. Zu den für die C1 und C2 Promotoren spezifischen cis-Elementen zählt eine vierfache Wiederholung der GATA-Box an den Positionen 812-815, 820-823, 832-835 und 838-841 im C1 Promotor und bei den Positionen 2739-2742, 2747-2750, 2759-2762 und 2765-2768 des C2 Promotors. Die GATA-Box wurde im 35S Promotor als Bindungsstelle des ASF-2 Transkriptionsfaktors identifiziert und liegt in dreifacher Wiederholung in dem licht-regulierten Promotor des Chlorophyll a/b Bindungsproteins der Petunie vor (Lam and Chua, 1989). An Position 832-837 des C1 Promotors und an Position 2759-2764 des C2 Promotors liegt eine l-Box mit der Sequenz GATAAG. Die l-Box wurde erstmals für die licht-regulierten rbcS Promotoren aus Tomate und Arabidopsis beschrieben (Giuliano al., 1988). An den Positionen 844-849 und 985-990 des C1 Promotors und den Positionen 2771-2776 und 2912-2917 des C2 Promotors befindet sich eine zweifache Wiederholung der GT-1 Bindungsstelle. Die GT-1 Bindungsstelle mit der Konsensussequenz G(A/G)(A/T)AA(A/T) wurde für die Promotoren zahlreicher lichtinduzierte Gene sowie dem Pathogenabwehrgen PR-1 beschrieben (Zhou, 1999). Eine zweifache Wiederholung einer für circadiane Expression wichtigen DNA-Sequenz befindet sich an den Positionen 913-922 und 1014-1023 im C1 Promotor und an den Positionen 2840-2849 und 2941-2950 des C2 Promotors. Die circadiane Box mit der Konsensus-sequenz CAANNNNATC (SEQ ID NO: 11) wurde in einem lichtregulierten Lhc Promotor der Tomate identifiziert (Piechulla et al., 1998).

### Veränderung des Kohlenhydratmetabolismus von Pflanzen durch Nutzung der wurzelspezifischen Promotoren 2-1-48 bzw. 2-1-36 am Beispiel der Vermeidung von Speicherstoffverlusten

Der Kohlenhydratmetabolismus von Pflanzen kann durch die Verwendung der wurzelspezifischen Promotoren der Gene 2-1-48 bzw. 2-1-36 gezielt verbessert werden. Als Beispiel wird die Expression des Invertaseinhibitorgens aus Tabak (Greiner et al., 1998) in der Wurzel von Zuckerrüben unter der Kontrolle der wurzelspezifisch aktiven Promotoren 2-1-48 bzw. 2-1-36 beschrieben. Durch die wurzelspezifische Expression werden die nach der Rübenernte bis zur Rübenverarbeitung auftretenden Verluste der Speichersubstanz Sucrose und die zuckertechnisch unerwünschte Akkumulation von Glucose und Fructose reduziert und die Zuckerausbeute dadurch insgesamt verbessert. Die Nutzung eines wurzelspezifischen Promotors erlaubt es im Vergleich zu einem konstitutiv in allen Geweben aktiven Promotor, die Expression des Invertaseinhibitorgens auf die Wurzel zu beschränken. Durch diese räumliche Beschränkung werden unerwünschte, ertrags-mindernde Effekte, die eine Expression des Inhibitorgens in allen Pflanzenteilen haben würde, vermieden.

Der Promotor 2-1-48 bzw. 2-1-36 kann als translationelle oder transkriptionelle Fusion mit dem Tabak-Invertaseinhibitorgen verknüpft und durch *A. tumefaciens* vermittelte Transformation in die Zuckerrübe transformiert werden. Zu diesem Zweck werden die entsprechenden binären Vektoren nach An (1987) in das *A. tumefaciens* Isolat C58 ATHV transformiert. Das pflanzliche Ausgangsmaterial für die Transformationen der Zuckerrüben sind Keimlinge. Dazu werden Samen der Zuckerrübe mit 12% Natriumhypochlorid oberflächendesinfiziert und anschließend 3-4 Wochen unter sterilen Bedingungen angekeimt. Die Keimblätter dieser Keimlinge werden dann mit Hilfe eines Skalpells klein geschnitten und für 5-10 min in einer verdünnten Übernachtkultur der *A. tumefaciens* Isolate (OD 0.2-0.3) inkubiert. Anschließend werden die Pflanzenteile trockengetupft und für 3 Tage auf festem 1/10 MS-Medium + 30 g/l Sucrose cokultiviert. Nach der Cokultivierungsphase werden die Explantate auf Selektionsmedium (MS-Medium + 0.25 mg/ BAP, 0.05 mg/l NAA) 500 mg/ Betabactyl, 20 g/L Sucrose) überführt. Zur Selektion der Transformanten wird Kanamycin verwendet. Nach 7-12 Wochen entstehen transgene Pflanzen, die vermehrt und bewurzelt werden.

Um den transgenen Charakter der Pflanzen molekularbiologisch nachzuweisen, wird das in die Pflanzen übertragene *npt*II Gen wie beschrieben unter Verwendung zweier genspezifischer Primer nachgewiesen. Die Expression des Invertaseinhibitorgens in den Zuckerrübenwurzeln wird durch RNA-Blot Studien nachgewiesen. Dazu werden Klonpflanzen ausgehend von den Primärtransformanten sowie nicht transgenen Kontrollpflanzen produziert und für die weitere Kultivierung in das Gewächshaus überführt. RNA wird nach Logemann et al. 1987 aus Blättern, Petiolen und der Wurzel der Invertaseinhibitor- und der Kontrollpflanzen isoliert, wie bereits geschildert gelelektrophoretisch aufgetrennt und auf eine Nylonmembran übertragen. Die anschließende Hybridisierung mit dem Invertaseinhibitorgen aus Tabak zeigt, dass das Gen ausschließlich in der Wurzel und nicht in den oberirdischen Organen der transgenen Pflanzen exprimiert wird. Um die positive Wirkung der wurzelspezifischen Expression zu analysieren wird ein Lagerungsversuch durchgeführt.

Die vollentwickelten Speicherwurzeln von 24 Wochen alten Inhibitor- und Kontrollpflanzen werden geerntet und durch eine 30 sekündige Behandlung in einem handelsüblichen Betonmischer (Attika) oberflächlich verletzt, um die für eine mechanische Rübenernte typischen Verletzungen zu erzeugen. Anschließend werden die Rüben bei 17° und 27°C eingelagert. Aus den bei 17°C gelagerten Pflanzenmaterial werden 1, 3, 4, 7, 14, 21, 28, 35 und 46 Tage nach der Ernte und bei bei 26°C gelagerten Rüben 1, 3, 4, 7, und 14 Tage nach der Ernte jeweils 5 Rüben entnommen. Die Rüben werden homogenisiert und der Gehalt an Fructose, Glucose und Sucrose wird bestimmt. Die bei 17°C und 27°C gelagerten nicht transgenen Zuckerrüben zeigen ab den 4. Lagerungstag eine deutliche Zunahme des Glucose- und Fructosegehaltes und eine Abnahme der Sucrosekonzentration. Die Speicherwurzeln der Invertaseinhibitorpflanzen hingegen weisen eine im Vergleich zu den Kontrollpflanzen geringere Akkumulation an Glucose- und Fructose und eine geringere Abnahme der Sucrosekonzentration auf.

### Veränderung des Kohlenhydratmetabolismus von transgenen Wurzeln durch Verwendung der wurzelspezifischen Promotoren 2-1-48 bzw. 2-1-36 am Beispiel der Reduzierung der wundinduzierten vakuolären Invertaseaktivität

Die beschriebene Verbesserung des Kohlenhydratstoffwechsels von Pflanzen kann auch an Hand transgener Wurzelkulturen ("hairy root") der Zuckerrübe nachgewiesen werden. Drei *A. tumefaciens* C58 ATHV Derivate, die mit dem 2-1-48 Promotor-Inhibitorkonstrukt, dem 2-1-36 Promotor-Inhibitorkonstrukt oder nur mit dem Ausgangsvektor transformiert worden sind, werden 24 h in flüssigem LB-Medium + 50 mg/l Kanamycin angezogen. Parallel wird der *Agrobacterium rhizogenes* Stamm 15834 in flüssigem TSB-Medium + 25 mg/l Rifampicin kultiviert. Anschließend werden die *A. tumefaciens* und der *A. rhizogenes* Stamm 21 h im jeweiligen Medium ohne Antibiotika angezogen. Die optische Dichte der Bakterienkulturen wird bestimmt und auf 0.4-0.6 A₆₀₀ eingestellt. Blattstiele von 3-4 Wochen alten Zuckerrüben, die unter *in vitro* Bedingungen angezogen wurden, werden in 0.5 cm lange Stücke geschnitten und kurz in eine 1:1 Mischung der *A. tumefaciens* und *A. rhizogenes* Kulturen getaucht. Die Blattstücke werden für 2 Tage bei Dauerlicht und 25°C auf festem MS-Medium + 0.5 mg/l BAP mit den Bakterien cokultiviert. Nach der Cokultivation werden die Stengelsegmente auf festem MS-Medium + 0.5 mg/l BAP + 350 mg/l Betabactyl (SmithKlineBeecham)+ 150-300 mg/l Kanamycin überführt und bei Schwachlicht kultiviert. Nach ca. 12 Tagen werden die ersten transgenen Wurzeln sichtbar, die abgetrennt und auf ½ B5-Medium + 300 mg/l Betabactyl + 300 mg /I Kanamycin vermehrt werden. Die transgenen Wurzelkulturen werden für weitere Experimente auf ½ B5-Medium ohne Antibiotika propagiert.

Die Expression des Invertaseinhibitorgens aus Tabak in den transgenen Wurzelkulturen wird durch ein RNA Blot Experiment nachgewiesen. RNA wird aus den Wurzelkulturen, die mit dem 2-1-48 Promotor-Inhibitorkonstrukt, dem 2-1-36 Promotor-Inhibitorkonstrukt bzw. mit dem Ausgangsvektor transformiert wurden, isoliert. Die RNA wird gelelektrophoretisch aufgetrennt, geblottet und der Nylon-Filter mit dem radioaktiv markierten Invertaseinhibitorgen als Sonde hybridisiert. Das Hybridisierungsergebnis zeigt, daß das Invertaseinhibitorgen des Tabaks nur in den mit dem 2-1-48 Promotor-Inhibitorkonstrukt und dem 2-1-36 Promotor-Inhibitorkonstrukt transformierten Wurzelkulturen nicht jedoch in den zu Kontrollzwecken mit dem Ausgangsvektor transformierten Wurzelkulturen exprimiert wird.

Die Verbesserung des Kohlenhydratstoffwechsels der Wurzelkulturen wird durch eine Bestimmung der wundinduzierbaren vakuolären Invertaseaktivität belegt. Die saure, vakuoläre Invertase der Zuckerrübe ist wie die Sucrose in der Vakuole lokalisiert. Während der ersten 12 Tage nach der Rübenernte wurde eine Zunahme der Invertzuckerkonzentration und der Aktivität der sauren, vakuolären Invertase beobachtet. Die durch das Köpfen der Zuckerrübe und dem anschließenden Erntevorgang auftretende Verletzung des Rübenkörpers sowie die Unterbrechung des Wachstumsphase gelten als Ursache für die Induktion der sauren Invertaseaktivitäten und damit dem Anstieg des Invertzuckergehaltes in der Rübe (Berghall et al., 1997).

Durch die wurzelspezifische Expression des Tabak-Invertaseinhibitorgens in den transgenen Wurzelkulturen wird die Aktivität der vakuolären Invertase in Reaktion auf den Wundreiz stark reduziert und die Invertzuckerbildung drastisch gemindert. Wurzelkulturen, die mit dem 2-1-48 Promotor-Inhibitorkonstrukt, dem 2-1-36 Promotor-Inhibitorkonstrukt bzw. mit dem Ausgangsvektor transformiert wurden, werden mit einem Skalpell in 3 mm lange Stücke geschnitten und 24 bzw. 48 h in flüssigem ½ B5-Medium inkubiert. Anschließend werden die Wurzeln homogenisiert und die Aktivität an saurer, vakuolärer Invertase bestimmt. Die Kulturen, in denen der Inhibitor durch die Promotoren 2-1-48 und 2-1-36 wurzelspezifisch exprimiert wird, zeigen in Reaktion auf eine Verwundung im Vergleich zu den Kontrollansätzen eine deutlich geringere Aktivität an saurer, vakuolärer Invertase und damit einen verbesserten Kohlenhydratstoffwechsel.

### Veränderung des Kohlenhydratmetabolismus von Pflanzen durch Nutzuncl der wurzelspezifischen Promotoren 2-1-48 bzw. 2-1-36 am Beispiel der Synthese neuer Kohlenhydrate

Der Kohlenhydratmetabolismus von Pflanzen kann durch die Verwendung der wurzelspezifischen Promotoren der Gene 2-1-48 bzw. 2-1-36 durch die Produktion neuer Kohlenhydrate gezielt verbessert werden. Als Beispiel wird die Synthese des Zuckeraustauschstoffes Palatinose und des Süßstoffes Palatinit in der Wurzel von Zuckerrüben unter der Kontrolle der wurzelspezifisch aktiven Promotoren 2-1-48 bzw. 2-1-36 beschrieben.

Palatinit (Glucosyl-α-(1,6)-sorbit/mannit) kann ausgehend von Sucrose in zwei Reaktionsschritten gebildet werden. Saccharose-6-glucosylmutase katalysiert die Umwandlung von Sucrose in Palatinose (Isomaltulose). Palatinose wird durch Sorbit-Dehydrogenase zu Palatinit (Isomalt) reduziert.

Die Kombination der wurzelspezifischen Promotoren 2-1-48 bzw. 2-1-36 mit einer Fusion zwischen dem vakuolären Transitpeptid des Palatingens der Kartoffel und dem Gen der Saccharose-6-glucosylmutase aus *Pseudomonas mesoacidophila* oder *Protaminobacter rubrum* (Klein, 1995) erlaubt es transgene Pflanzen zu produzieren, die Palatinose spezifisch nur in den Wurzeln produzieren. Diese Konstrukte werden wie zuvor beschrieben in Pflanzen wie die Zuckerrübe, die die Speichersubstanz Sucrose in den Vakuolen der Wurzel einlagert, transformiert. Als Selektionsmarker wird das *npt*II Gen in Kombination mit dem Antibiotikum Kanamycin verwendet. Der transgene Charakter der durch die Kanamycin Selektion identifizierten Zuckerrüben wird durch PCR unter Verwendung der für das *npt*II-Gen spezifischen Primer verifiziert.

Die Konzentration an Palatinose in den Wurzeln verschiedener Transformanten wird durch HPLC bestimmt. Die Proben können durch Einsatz des Laufmittels 0.1 M NaOH über eine Hamilton RCX-10 (250 x 4,1 mm) Säule aufgetrennt werden. Die Quantifizierung der Palatinose in den Wurzelextrakten erfolgt mit Bezug auf Palatinose-Referenzen bekannter Konzentration. Unter Nutzung dieser analytischen Technik können Zuckerrüben Transformanten identifiziert werden, die Palatinose in den Wurzeln produzieren.

Für die Herstellung von Palatinit produzierenden Pflanzen werden die Palatinose-Zuckerrüben ausgewählt, die die höchste Konzentration an Palatinose in der Wurzel akkumulieren. Diese Transformanten werden ein weiteres Mal mit einem Konstrukt transformiert, dass den 2-1-48 bzw. 2-1-36 Promotor in Kombination mit dem Sorbit-Dehydrogenasegen enthält. Das Dehydrogenasegen ist dabei zum einen mit einer vakuolären Transitsequenz fusioniert bzw. frei von einer Signalsequenz, so dass das Genprodukt im Cytoplasma lokalisiert wird. Für die Selektion der Doppeltransformanten wird ein binärer Vektor verwendet, der entweder das pat- oder das CP4 Gen enthält, so daß Basta oder Roundup als Selektionsmittel verwendet werden können. Transformanten, die eine entsprechende Herbizidresistenz aufweisen werden zusätzlich molekularbiologisch charakterisiert, indem die Anwesenheit des bar bzw. CP4 Gens durch PCR nachgewiesen wird.

Die Synthese von Palatinit in den transgenen Pflanzen wird durch HPLC quantitativ nachgewiesen. Diese Untersuchungen zeigen, dass die Coexpression eines bakteriellen Saccharose-6-glucosylmutase Gens in Kombination mit einem Sorbit-Dehydrogenase Gens unter Verwendung von wurzelspezifischen Promotoren zur Bildung von Palatinit führt. Erst die Nutzung der wurzelspezifischen Promotoren erlaubt es im Vergleich zu konstitutiven Promotoren Transformanten zu erhalten, die bei einem normalen Phänotyp ohne unerwünschte physiologische Veränderungen Palatinit in wirtschaftlich interessanten Konzentrationen produzieren.

### Veränderung des Kohlenhvdratmetabolismus von transgenen Wurzeln durch Verwendung derwurzelspezifischen Promotoren 2-1-48 bzw. 2-1-36 am Beispiel der Synthese neuer Kohlenhydrate

Eine Verbesserung des Kohlenhydratstoffwechsels von Pflanzen durch die Synthese neuer Kohlenhydrate kann auch an Hand transgener Wurzelkulturen ("hairy root") der Zuckerrübe nachgewiesen werden. Zu diesem Zweck werden die zuvor beschriebenen Expressionskassetten bestehend aus dem 2-1-48 bzw. 2-1-36 Promotor und der Fusion zwischen der Transitsequenz des Patatingens mit dem Gen der Saccharose-6-glucosylmutase aus *Pseudomonas mesoacidophila* bzw. *Protaminbacterrubrum* und dem 2-1-48 bzw. 2-1-36 Promotor und dem Sorbeit-Dehydrogenasegen in den binären Vektor BIN19 (Clontech, Heidelberg, Deutschland) integriert. Die resultierenden Konstrukte werden in den *A. tumefaciens* Stamm C58 ATHV transformiert. Durch eine Cotransformation mit dem *A. rhizogenes* Stamm 15834 wird die Genkombination aus Saccharose-6-glucosylmutase und Sorbit Dehydrogenase wie beschrieben in transgene Zuckerrübenwurzeln transformiert.

Der quantitative Nachweis von Palatinit geschieht wie dargestellt durch HPLC. Diese analytischen Untersuchungen zeigen, daß die Expression des Saccharose-6-glucosylmutase- und des Sorbit-Dehydrogenasegens unter der Kontrolle der wurzelspezifischen Promotoren 2-1-48 und 2-1-36 zur Bildung des neuen Kohlenhydrates Palatinit in den transgenen Wurzelkulturen führt.

### Veränderung des Kohlenhydratmetabolismus von Pflanzen durch Nutzung der wurzelspezifischen Promotoren 2-1-48 bzw. 2-1-36 am Beispiel der Synthese neuartige Polymere

Der Kohlenhydratmetabolismus von Pflanzen kann durch die Verwendung der wurzelspezifischen Promotoren der Gene 2-1-48 bzw. 2-1-36 durch die Produktion neuer Polymere gezielt verbessert werden.

Die Bildung neuer Polymere kann beispielhaft in Wurzeln von Zuckerrüben durch die Expression einer Fructan-Fructan-Fructosyltransferase, einer Sucrose-Sucrose-Fructosyltransferase, einer Levansucrase, einer Sucrose-Fructan-6-Fructosyltransferase und einer Fructosyltransferase erfolgen.
Die codierenden Bereiche der Enzyme werden jeweils mit dem wurzelspezifischen Promotor des Gens 2-1-48 bzw. 2-1-36 verknüpft und nach bekannter Technik mit Hilfe von A. tumefaciens in Zuckerrüben transformiert bzw. durch Cotransformation mit *A. rhizogenes* in transgene Wurzelkulturen übertragen. Die Expression der transferierten Gene wird zum einen durch RNA-Blot Studien unter Verwendung der codierenden Bereiche als Hybridisierungssonden und zum anderen durch enzymatische Aktivitätsmessungen nachgewiesen. Durch zuckeranalytische Untersuchungen können die Transformaten identifiziert werden, die die gewünschten Polymere in höchster Konzentration enthalten.

### Veränderung des Stickstoffmetabolismus von Pflanzen durch Nutzung der wurzelspezifischen Promotoren 2-1-48 bzw. 2-1-36 und der in oberirdischen Organen aktiven C1 bzw. C2 Promotoren

Der Stickstoffmetabolismus von Pflanzen kann durch die Verwendung der wurzelspezifischen Promotoren der Gene 2-1-48 bzw. 2-1-36 und durch die Nutzung der in oberirdischen Organen aktiven C1 bzw. C2 Promotoren in vielfältiger Hinsicht verbessert werden. Die wurzel- bzw. blattspezifische Erhöhung der Zahl ge-eigneter Transportproteine verbessert die Aufnahme und den Transport von N-Ver-bindungen in der Pflanze.

Durch die wurzelspezifische Expression von Transportproteingenen für Ammonium-(NH₄⁺), Nitrat- (NO₃⁻) und Nitrit (NO₂⁻)-Ionen kann die Stickstoffaufnahme aus dem Boden gesteigert und die Nutzung von N-Dünger verbessert werden. Einer effizienteren Nutzung der in der Wurzel bereits aufgenommenen N-Verbindungen dient die blattspezifische Expression von Nitrat- und Nitrittransportproteinen durch Verwendung der in oberirdischen Organen aktiven Promotoren C1 bzw. C2. Die blattspezifische Expression von Nitrattransportproteinen führt zu einer verstärkten Phloementladung der Nitrationen und zu einer höheren Nitrataufnahme in den Blattparenchymzellen. Durch die erhöhte Nitrat-Akkumulation in den Blattparenchymzellen wird die N-Reduktion in den Plastiden verstärkt. Der erhöhte Transport von Nitrit aus dem Cytosol in die Plastiden durch die blattspezifische Expression von geeigneten Nitrittransportproteinen führt ebenfalls zu einer stärkeren Aminosäurebiosynthese.

### Erhöhung der Toleranz gegenüber Pathogen durch Nutzung der wurzelspezifischen Promotoren 2-1-48 bzw. 2-1-36 und/oder der in oberirdischen Organen aktiven Promotoren C1 bzw. C2.

Die wurzelspezifischen Promotoren 2-1-48 bzw. 2-1-36 und die in oberirdischen Organen aktiven Promotoren C1 und C2 können für die Ausprägung von Merkmalen genutzt werden, die die Resistenz bzw. Toleranz gegenüber Pathogenen verbessern.

### Erhöhung der Toleranz gegenüber phytopathogenen Viren durch Nutzung der wurzelspezifischen Promotoren 2-1-48 bzw. 2-1-36 und/oder der in oberirdischen Organen aktiven Promotoren C1 bzw. C2.

Zahlreiche phytopathogene Viren der Zuckerrübe zeigen eine Organspezifität, d.h. die virale Vermehrung erfolgt gewöhnlich nicht in der ganzen Pflanze sondern nur in einem bestimmten Organ oder Gewebetyp. Ebenso sind die Schäden, die die virale Infektion hervorruft, in der Regel auf das befallene Organ beschränkt. Virale Krankheitserreger der Zuckerrübe mit einer Organspezifität sind z. B. BNYW mit der Präferenz für die Wurzel und BMYV und BYV mit der Beschränkung auf Rübenblätter.

Die wurzelspezifischen Promotoren 2-1-48 bzw. 2-1-36 können genutzt werden, um eine wurzelspezifische BNYVV Resistenz in der Zuckerrübe zu entwickeln. Dazu wird für die Umsetzung der gene silencing abhängigen Virusresistenzstrategie eine native oder mutagenisierte DNA-Teilsequenz des viralen BNYVV Genoms mit dem 2-1-48 oder 2-1-36 Promotor kombiniert. Die Kombination zwischen der Promotorsequenz und der viralen DNA-Sequenz wird so gestaltet, daß die Transkription der BNYVV Sequenz zu einem gegen das BNYVV wirksamen gene silencing führt. Die Wirksamkeit des Ansatzes wird über eine Bestimmung des Virustiters in den Pflanzen unter Nutzung eines ELISA Tests, der gegen das Hüllprotein des BNYW gerichtet ist, bestimmt.

### Erhöhung der Toleranz gegenüber - engen Nematoden durch Nutzung der wurzelspezifischen Promotoren 2-1-48 bzw. 2-1-36

Die wurzelspezifische Aktivität der Promotoren 2-1-48 bzw. 2-1-36 kann genutzt werden, um in Pflanzen wie der Zuckerrübe eine Resistenz gegenüber Nematoden wie *Heterodera schachtii* oder in Kartoffeln eine Resistenz gegenüber *Globodera pallida* oder *Globodera rostochiensis* auszuprägen.

Zu diesem Zweck wird ein Nematodenresistenzgen oder ein Gen für eine nematozid wirkende Komponente mit dem Promotor 2-1-48 bzw. 2-1-36 translationell oder transkriptionell fusioniert und in einen binären Pflanzentransformationsvektor wie z.B. BIN19 inseriert. Nematodenresistenzgene, die eine Resistenz gegenüber *Heterodera schachtii* vermitteln, sind das Hs1^{pro-1} aus *Beta procumbens* (Cai et al., 1997) und im Fall von *Globodera pallida* das Gpa2-Gen der Kartoffel (Van der Vossen et al., 2000).

Durch *A. tumefaciens* vermittelte Transformation werden die Promotorgenkombinationen in der bereits beschriebenen Weise für die Gewinnung transgener Zuckerrüben oder Kartoffeln genutzt. Weiterhin können die Genkonstrukte durch eine Cotransformation unter Verwendung von *A. tumefaciens* und *A. rhizogenes* entsprechend dem dargestellten Protokoll in transgene Wurzelkulturen der Zuckerrübe überführt werden. Die Transgenität der produzierten Pflanzen wird molekularbiologisch über PCR durch Amplifikation des nptll-Gens nachgewiesen. Die wurzelspezifische Expression des resistenzvermittelnden Faktors, wie z. B. dem Hs1^{pro-1} Gen, wird durch eine RNA Blot Studie unter Einsatz des Hs1^{pro-1} Gens als Hybridisierungssonde belegt. Die Resistenz der transgenen Pflanzen bzw. transgenen Wurzelkulturen wird durch einen Nematoden-Resistenztest geprüft und nachgewiesen. Die Durchführung der Nematoden-Resistenzprüfung mit *H. schachtii* an transgenen Wurzelkulturen der Zuckerrübe ist bei Cai et al. (1997) beschrieben. Die experimentelle Beschreibung der Resistenzprüfung von *in-vitro* Karatoffeln gegenüber *G. pallida* bzw. den Verweis auf die Durchführung von Gewächshausprüfungen findet der Fachmann bei (Van der Vossen et al., 2000).

Die Vorzüge der wurzelspezifischen Expression des Nematodenresistenzgens bzw. der nematoziden Komponente liegt neben der hohen Resistenz in der Tatsache, daß das resistenzvermittelnde Genprodukt nur in dem zu schützenden Organ gebildet wird. Die Abwesenheit des resistenzvermittelnden Genprodukts in Pflanzenteilen, die wie die Kartoffelknolle für den Konsum bestimmt sind, erhöht die gesellschaftliche Akzeptanz und damit die Absatzchance der transgenen Pflanze und des daraus gewonnenen Produktes.

### Erhöhung der Toleranz transgener Pflanzen gegenüber phytopathogenen Pilzen durch Nutzung der wurzelspezifischen Promotoren 2-1-48 bzw. 2-1-36 und/oder der in oberirdischen Organen aktiven Promotoren C1 bzw. C2.

Die wurzelspezifischen Promotoren 2-1-48 bzw. 2-1-36 und die in oberirdischen Organen aktiven Promotoren C1 bzw. C2 können genutzt werden, um in Kombination mit einem Gen oder einer Genkombination eine direkte oder indirekte antifungale Wirkung in Pflanzen auszuprägen. Die antifungale Wirkung führt zu dem Merkmal der erhöhten Pilzresistenz oder Pilztoleranz.

Die wurzelspezifischen Promotoren 2-1-48 bzw. 2-1-36 und die in oberirdischen Organen aktiven Promotoren C1 bzw. C2 werden mit Genen der Pathogenabwehr, deren Genprodukte eine direkte antifungale Wirkung haben, translationell oder transkriptionell fusioniert. Die Promotorgenkombinationen werden in den binären Transformationsvektor BIN19 kloniert und durch *A. tumefaciens* vermittelte Transformation in Zuckerrübe, Kartoffel und Raps transformiert. Die Transgenität der Pflanzen wird wie beschrieben durch PCR überprüft und die Expression der Gene in den Wurzeln oder Blättern durch RNA Blot Studien verifiziert. Die erhöhte Pilzresistenz der Pflanzen wird bei Pilzresistenzprüfungen, wie sie nachfolgend beispielhaft für Resistenzprüfung der Zuckerrüben gegenüber *Cercospora beticola* beschrieben wird, beobachtet.

Für die Infektion von Zuckerrüben mit dem Blattfleckenerreger *C*. *beticola* werden neben den transgenen Pflanzen Zuckerrüben des toleranten Genotyps 1 K0088 und des anfälligen Genotyps 3S0057 unter Gewächshausbedingungen angezogen. Zwei Wochen vor der geplanten Inokulation werden 20 V8-Gemüsesaftplatten (40% Albani-Gemüsesaft) mit vier verschiedenen *C. beticola* Isolaten beimpft und bei 25°C inkubiert. Unmittelbar vor der Inokulation wird der pilzbewachsene Agar zusammen mit 0,5 I Wasser in einem Hochleistungsrührwerk (UM5 Universal, Stephan) homogenisiert. Die Konzentration an Myzelfragmenten und Pilzsporen in dem Homogenat wird mit Hilfe einer Zählkammer bestimmt. Die Inokulumdichte wird durch Verdünnen mit Wasser auf eine Konzentration von 100.000 Fragmente/ml eingestellt. Das verdünnte Homogenat wird mit Hilfe einer Rückenspritze (Gloria 176T) auf die 12 Wochen alten Zuckerrüben gesprüht. Zu Kontrollzwecken werden Pflanzen mit einem pilzfreiem Agarhomogenat besprüht. Die Pflanzen werden nach der Inokulation für 4 Tage bei 25°C und 95% Luftfeuchtigkeit in einem Gewächshaus inkubiert. Nach dem vierten Tag wird die Luftfeuchtigkeit auf 60-70 % reduziert. Vierzehn und einundzwanzig Tage nach der Inokulation wird der Befall der Blätter von den pilz- und agar-inokulierten Pflanzen optisch bewertet.

Die transgenen Zuckerrüben zeigen z. B. im Fall der Nutzung der in oberirdischen Organen aktiven Promotoren neben einer erhöhten Resistenz gegenüber den Blattparasiten *Cercospora beticola* auch eine erhöhte Resistenz gegenüber *Ramularia beticola* und *Erysiphe betae.* Zuckerrüben, bei denen die Pathogenabwehrgene wurzelspezifisch exprimiert werden, weisen eine erhöhte Resistenz gegenüber den Wurzelparasiten *Rhizoctonia solani* und *Aphanomyces cochlioides* auf.

Überraschenderweise führt die organspezifische, konstitutive Expression von PathogenAbwehrgenen nicht zu der bei einer konstitutiven Expression in der gesamten Pflanze oft beobachteten Kleinwüchsigkeit oder Ertragsminderung. Ein weiterer Vorteil der wurzel- bzw. für oberirdischen Organen spezifischen Genexpression ist, daß die resistenzvermittelnden Genprodukte nur in dem zu schützenden Organ gebildet werden.

Gene, deren Genprodukte eine indirekte antifungale Wirkung haben, benötigen eine besonders sorgfältige Expressionskontrolle, um negative Folgen einer ungewollten Aktivierung der pflanzlichen Abwehr zu vermeiden. Ein Beispiel für eine indirekte antifungale Wirkung ist der Effekt, der von der Coexpression eines pflanzlichen Resistenz-(R)-Gens in Kombination mit einem Avirulenz-(avr)-Gen in einer pflanzlichen Zelle ausgeht. Die gemeinsame Expression von R- und avr-Gen führt zu einer intensiven Aktivierung der pflanzlichen Abwehr und bedarf einer strikten Regulation. Die Regulation wird erreicht, indem entweder das R-Gen oder/und das avr-Gen unter die Kontrolle eines pathogeninduzierbaren Promotors gestellt wird.

Pathogeninduzierbare Promotoren, wie z. B. der Vst1-Promotor aus dem Wein, zeigen in den oberirdischen Organen transgener Pflanzen neben einer lokalen, spezifischen Aktivierung nach Befall durch wirtschaftlich relevante phytopathogene Pilze häufig eine unspezifische Aktivierung im Wurzelbereich (Stahl et al., 1995). Diese Promotoraktivierung wird durch pythopathologisch unbedenkliche Mikroorganismen hervorgerufen und macht Promotoren wie den Vst1 ungeeignet für die Expression eines R- oder avr-Gens. Durch eine organ- oder gewebespezifische, konstitutive Expression des R- oder des avr-Gens unter der Kontrolle der für oberirdischen Organen spezifischen Promotoren C1 bzw. C2 kann ein pathogenaktivierbarer Promotor wie z.B. der Vst1 Promotor für die Expression des korrespondierenden avr- oder R-Gens jedoch genutzt werden. Das R/avr-Genkonzept kann so organspezifisch umgesetzt werden.
Um eine Nutzung des R/avr-Genkonzeptes in den oberirdischen Organen von Zuckerrübe, Kartoffel, Raps und *Arabidopsis thaliana* zu erreichen, wird das entsprechende R-Gen mit dem Promotor C1 bzw. C2 fusioniert. Im Fall der Kartoffel wird als R-Gen z. B. das Cf-9 Gen der Tomate verwendet und für Raps und *A. thaliana* das RPM1 aus *A*. *thaliana.* Das korrespondierende avr-Gen (*avr*9 aus *Cladosporium fulvum* für Kartoffel und *avr*B aus *Pseudomonas* spp. für Raps bzw. A. thaliana) wird transkriptionell oder translationell mit einem pathogensensitiven Promotor wie dem *Vst*1-, dem *hcbt*2 oder mit einem der chimären pathogensensitiven Promotor (PCT/EP99/08710, Chimeric promoters capable of mediating gene expression in plants upon pathogen infection and uses thereof) kombiniert. Die zwei Expressionskassetten werden in einem binären Vektor wie BIN19 integriert. Durch *A. tumefaciens* vermittelte Transformation werden die C1/C2 Promotor-R-Gen-Kombinationen und die Fusion zwischen pathogensensitiven Promotor und avr-Gen in Zuckerrübe, Kartoffel, Raps und *Arabidopsis thaliana* transformiert.
Die nachfolgenden Resistenzprüfungen zeigen, daß die mit der R/avr-Genkombination unter Verwendung des C1 bzw. C2 Promotors transformierten Zuckerrüben eine sehr hohe Resistenz gegenüber den Blattparasiten Cercospora *beticola, Ramularia beticola* und *Erysiphe betae* aufweisen. Resistenzprüfungen der transgenen Kartoffeln zeigen eine sehr hohe Resistenz gegenüber *Phytophthora infestans* und bei den Rapspflanzen wird eine hohe Resistenz gegenüber *Phoma lingam, Sclerotinia sclerotiorum* und *Cylindro-sporium* concentricum beobachtet. Die transgenen *A. thaliana* zeigen eine Resistenz gegenüber *Peronospora parasitica.* Allen transgenen Pflanzen gemeinsam ist, daß im Wurzelbereich keine unerwünschten Nekrotisierungen oder Fehlbildungen auftreten, die auf eine unerwünschte Aktivierung des R/avr-Systems hinweisen.

### Repression der Pathogenabwehr in nicht befallenen Geweben durch Verwendung dewurzelspezifischen Promotoren 2-1-48 bzw. 2-1-36 oder in oberirdischen Organen aktiven Promotoren C1 und C2

Eine Aktivierung der Pathogenabwehr in nichtbefallenen Geweben kann aufgrund der damit verbundenen Konsequenzen wie Zelltod und Umstellung des Zellstoffwechsels negative Folgen für die Pflanzenentwicklung und den Ertrag haben. Auslöser für die unerwünschte Aktivierung kann die Verwendung von pathogensensitiven Promotoren nicht ausreichender Spezifität in Kombination mit Pathogenabwehrgenen sein. Ein Beispiel für dieses Problem ist die Verwendung von pathogensensitiven Promotoren für die Expression eines Avirulenzgens in Kombination mit einem korrespondierenden Resistenzgen.

Die wurzelspezifischen Promotoren können genutzt werden, um eine unerwünschte Aktivierung von z.B. pathogensensitiven Promotoren in der Wurzel zu unterdrücken. Dadurch können besagt Promotoren trotz "Hintergrundaktivität" für die Umsetzung des R/avr-Konzeptes genutzt werden.

**Tabelle 1:**

| Vergleich der Transkriptakkumulation des wurzelspezifisch exprimierten Gens 2-1-48 in verschiedenen Organen der Zuckerrübe | | | | | | |
|---|---|---|---|---|---|---|
| Gesamtzell-RNA wurde nach der Aussaat zu verschiedenen Entwicklungszeitpunkten (4, 6, 10 12, 16, 22 Wochen) aus "sink"- und "source"-Blättern, aus Petiolen, Pfahlwurzeln und Seitenwurzeln von Zuckerrüben isoliert und durch eine RNA-Blot Analyse untersucht. Als Hybridisierungssonde wurde das cDNA-Fragment 2-1-48 eingesetzt. Die durch die Promotoraktivität gebildete Transkriptmenge wurde mit Hilfe eines Phosphoimager quantifiziert und in der Tabelle für jeden Versuchszeitpunkt dargestellt. Die Hintergrundaktivität der Nylonfilter wurde für jeden Filter einmal bestimmt und von den Messwerten subtrahiert. Der Hintergrundwert beträgt bei der Analyse der Zeitpunkte 4-10 Woche 164,9 psI und bei den Zeitpunkten 12-22 Woche 215,7 psI. | | | | | | |

| Organ | 4 Wochen | 6 Wochen | 10 Wochen | 12 Wochen | 16 Wochen | 22 Wochen |
|---|---|---|---|---|---|---|
| "source" Blatt | 86,4^{1,4} | 53,6 | 34,9 | 0,7 | 86,5 | 19,1 |
| "sink" Blatt | 56,1² | n.b.³ | 22,1 | 34,0 | 30,8 | 8,2 |
| Petiole | n.b.³ | 74,7 | 49,4 | 72,0 | 36,0 | 16,5 |
| Pfahlwurzel | 2231,0 | 2506,0 | 2581,2 | 6082,8 | 1408,5 | 681,3 |
| Seitenwurzel | n.b.³ | 1633,0 | 486,8 | 4932,2 | 1403,5 | 833,1 |

| | | | | | | |
|---|---|---|---|---|---|---|
| ¹ Als "source" Blatt wurde bei den 4 Wochen alten Zuckerrüben das erste Blattpaar gewählt. ² Als "sink" Blatt wurden bei den 4 Wochen alten Zuckerrüben die Keimblätter ausgewählt. ³ n.b. = nicht bestimmt. ⁴ Messwerte sind in psI (photo stimulated luminescence)-Einheiten angegeben. | | | | | | |

**Tabelle 2 :**

| Vergleich der Transkriptakkumulation des wurzelspezifisch exprimierten Gens 2-1-36 in verschiedenen Organen der Zuckerrübe | | | | | | |
|---|---|---|---|---|---|---|
| Gesamtzell-RNA wurde nach der Aussaat zu verschiedenen Entwicklungszeitpunkten (4, 6, 10 12, 16, 22 Wochen) aus "sink"- und "source"-Blättern, Petiolen, Pfahlwurzeln und Seitenwurzeln von Zuckerrüben isoliert und durch eine RNA-Blot Analyse untersucht. Als Hybridisierungssonde wurde das cDNA-Fragment 2-1-36 eingesetzt. Die durch die Promotoraktivität gebildete Transkriptmenge wurde mit Hilfe eines Phosphoimager quantifiziert und in der Tabelle für jeden Versuchszeitpunkt dargestellt. Die Hintergrundaktivität der Nylonfilter wurde für jeden Filter einmal bestimmt und von den Messwerten subtrahiert. Die Hintergrundwerte betragen bei der Analyse der Zeitpunkte 4-10 Woche 145 psI und bei den Zeitpunkten 12-22 Woche 212,67 psI. | | | | | | |

| Organ | 4 Wochen | 6 Wochen | 10 Wochen | 12 Wochen | 16 Wochen | 22 Wochen |
|---|---|---|---|---|---|---|
| "source" Blatt | 3,4^{1,4} | 2,0 | 7,8 | 0 | 9,5 | 5,5 |
| "sink" Blatt | 0² | 4,8 | 7,3 | 3,6 | 12,4 | 11 |
| Petiole | n.b.³ | 9,8 | 4,7 | 5,3 | 15,4 | 13,7 |
| Pfahlwurzel | 67,2 | 102,7 | 136,2 | 803,3 | 546,4 | 518,7 |
| Seitenwurzel | n.b.³ | 71,5 | 177,4 | 888,0 | 363,1 | 874,0 |

| | | | | | | |
|---|---|---|---|---|---|---|
| ¹ Als "source" Blatt wurde bei den 4 Wochen alten Zuckerrüben das erste Blattpaar gewählt. ² Als "sink" Blatt wurden bei den 4 Wochen alten Zuckerrüben die Keimblätter ausgewählt. ³ n.b. = nicht bestimmt. ⁴ Messwerte sind in psI (photo stimulated luminescence)-Einheiten angegeben. | | | | | | |

**Tabelle 3:**

| Vergleich der Transkriptakkumulation des Gens 2-3-9 in verschiedenen Organen der Zuckerrübe | | | | | | |
|---|---|---|---|---|---|---|
| Gesamtzell-RNA wurde nach der Aussaat zu verschiedenen Entwicklungszeitpunkten (4, 6, 10 12, 16, 22 Wochen) aus "sink"- und "source"-Blättern, aus Petiolen, Pfahlwurzeln und Seitenwurzeln von Zuckerrüben isoliert und durch eine RNA-Blot Analyse untersucht. Als Hybridisierungssonde wurde das cDNA-Fragment 2-3-9 eingesetzt. Die durch die Promotoraktivität gebildete Transkriptmenge wurde mit Hilfe eines Phosphoimager quantifiziert und in der Tabelle für jeden Versuchszeitpunkt dargestellt. Die Hintergrundaktivität der Nylonfilter wurde jeweils an vier unabhängigen Stellen bestimmt und der Mittelwert des Hintergrundes von den Messwerten subtrahiert. Die Hintergrundwerte betragen bei der Analyse der Zeitpunkte 4-10 Woche im Mittel 155,1 psI (Einzelwerte: 150,7; 150,1; 141,7 und 177,9) und bei den Zeitpunkten 12-22 Woche im Mittel 155,9 psI (Einzelwerte: 149,3, 150,4; 178,3 und 145,8). | | | | | | |

| Organ | 4 Wochen | 6 Wochen | 10 Wochen | 12 Wochen | 16 Wochen | 22 Wochen |
|---|---|---|---|---|---|---|
| "source" Blatt | 3788^{1,4} | 2084,2 | 249,6 | 1117,9 | 2073,7 | 1634,4 |
| "sink" Blatt | 1927,7² | 2239,3 | 2067,7 | 3976,2 | 3471,2 | 4269,6 |
| Petiole | n.b.³ | 1237,2 | 960 | 1589,7 | 1140,6 | 774,1 |
| Pfahlwurzel | 0 | 14,2 | 0 | 17,8 | 27,3 | 13,2 |
| Seitenwurzel | n.b.³ | 0 | 0 | 19,4 | 26,5 | 0 |

| | | | | | | |
|---|---|---|---|---|---|---|
| ¹ Als "source" Blatt wurde bei den 4 Wochen alten Zuckerrüben das erste Blattpaar gewählt. ² Als "sink" Blatt wurden bei den 4 Wochen alten Zuckerrüben die Keimblätter ausgewählt. ³ n.b. = nicht bestimmt. ⁴ Messwerte sind in psI (photo stimulated luminescence)-Einheiten angegeben. | | | | | | |

**Tabelle 4:**

| Nachweis der C1 Promotoraktivität in Zuckerrübenblättern durch ballistische Transformation. | | | | | | | | |
|---|---|---|---|---|---|---|---|---|
| **Konstrukt** | **Blatttyp** | **Platte 1** | **Platte 2** | **Platte 3** | **Platte 1** | **Platte 2** | **Platte 3** | **Mittelwert** |
| | | Photinus /Renilla Luciferase Aktivität¹ | Photinus /Renilla Luciferase Aktivität¹ | Photinus /Renilla Luciferase Aktivität¹ | Normalsierte Genexpression² | Normalsierte Genexpression² | Normalsierte Genexpression² | Normalisierte Genexpression² |
| pc1L-xxx | klein | 8590 | 6294 | 24431 | 7,8 | 7,7 | 8,6 | 8,0 |
| | | 119120 | 91725 | 294751 | | | | |
| pc1L-xxx | groß | 16149 | 8078 | 9253 | 6,0 | 6,8 | 16,1 | 9,6 |
| | | 278403 | 131141 | 72079 | | | | |
| pc1L-7000 | klein | 4751 | 4811 | 6008 | 2,5 | 2,8 | 4,4 | 3,2 |
| | | 192866 | 175901 | 144327 | | | | |
| pc1L-7000 | groß | 13465 | 8571 | 1231 | 5,7 | 11,6 | 3,6 | 7,0 |
| | | 246870 | 87835 | 42334 | | | | |
| ohne DNA | klein | 228 | | | | | | |
| | | 12500 | | | | | | |
| ohne DNA | groß | 300 | | | | | | |
| | | 16369 | | | | | | |

| | | | | | | | | |
|---|---|---|---|---|---|---|---|---|
| ¹ Relative Lichteinheiten. ² Die normalisierten Expressionswerte wurden folgendermaßen berechnet: (*Photinus* Meßwert _{(Konstrukt)} - *Photinus* Meßwert _{(ohne DNA)} / *Renilla* Meßwert _{(Konstrukt)} - *Renilla* Meßwert _{(ohne DNA)} ) x 100. | | | | | | | | |

### Literatur:

Altschul, S.F. et al. (1990). Basic Local Alignment search tool, J.Mol. Biol. 215: 403-410
An, G. (1987). Binary Ti vectors für plant transformation and promoter analysis. Methods Enzymol. 153, 292-305.
Berghall, S., Briggs, S., Elsegood, S. E., Eronen, L., Kuusisto, J. O., Philip, E. J., Theobald, T. C., and Walliander, P. (1997). The role of sugar beet invertase related enzymes during growth, storage and processing. Zuckerind. 122, 520-530.
Cai, D. et al., (1997). Positional cloning of a gene for nematode resistance in sugar beet. Science 275, 832-834.
DE 4207358 A1 (Institut für Genbiologische Forschung Berlin GmbH). Expressionskassette und Plasmide zur schliesszellenspezifischen Expression und ihre Verwendung zur Herstellung transgener Pflanzenzellen und Pflanzen.
De Greve, H., Dhaese, P., Seurinck, J., Lemmers, M., Van Montagu, M., and Schell, J. (1982). Nucleotide sequence and transcript map of the Agrobacterium tumefaciens Ti plasmid-encoded Octopine synthase gene. J Mol. Appl. Genet. 1 (6), 499-511.
Depicker, A., Stachel, S., Dhaese, P. Zambryski, P., and Goodman, H. M. (1982). Nopaline synthase: Transcript mapping and DNA sequence. J Mol. Appl. Genet. 1 (6), 561-573.
Diatchenko, L., Lau, Y.-F. C., Campbell, A. P., Chenchik, A., Moqadam, F., Huang, B., Lukyanov, S., Lukyanov, K., Gurskaya, N., Sverdlov, E. D., and Siebert, P. D. (1996). Suppression subtractive hybridization: A method for generating differentially regulated or tissue-specific cDNA probes and libraries. Proc. Natl. Acad. Sci. USA 93, 6025-6030.
Dzelzkàlns, V. A., Thorsness, M. K., Dwyer, K. G., Baxter, J. S., Balent, M. A., Nasrallah, M. E., and Nasrallah, J. B. (1993). Distinct cis-acting elements direct pistil-specific and pollenspecific activity of the Brassica S locus glycoprotein gene promoter. Plant Cell 5, 855-863.
EP 0344029 B1. (Plant Genetic Systems, N. V. 1040 Brussel). Plants with modified stamen cells.
EP 99/08710, Chimeric promoters capable of mediating gene expression in plants upon pathogen infection and uses thereof.
Giuliano, G., Pichersky, E., Malik, V. S., Timko, M. P., Scolnik, P. A., and Cashmore, A. R. (1988). An evolutionarily conserved protein binding sequence upstream of a plant light regulated gene. Proc. Natl. Acad. Sci. USA 85, 7089-7093.
Greiner, S., Krausgrill, S., and Rausch, T. (1998). Cloning of a tobacco apoplasmic invertase inhibitor: Proof of function of the recombinant protein and expression analysis during plant development. Plant Physiol. 116, 733-742.
Hesse, H., and Willmitzer, L. (1996). Expression analysis of a sucrose synthase gene from sugar beet (Beta vulgaris L). Plant Mol Biol 30, 863-872.
Höfgen R. & Hesse H.: DE 19607697, erteilt 09-04-1998 / WO 97/32027,veröff. 04-09-199
Horsch, R. B., Fry, J. E., Hoffmann, N. L., Rogers, S. G., Fraley, R. T. (1985). A simple and general method for transferring genes into plants. Science 227, 1229-1231.
Klein. K. (1995). Die Bedeutung von Saccharose-Stoffwechselgenen für die bakterielle Isomaltulose-Herstellung. Dissertation Universistät Stuttgart.
Lam, E. and Chua, N. H. (1989). ASF-2: A factor that binds to the cauliflower mosaic virus 35S promoter and a conserved GATA motif in cab promoters. Plant Cell 1, 1147-1156.
Logemann, J., Schell, J., and Willmitzer, L. (1987). Improved method for the isolation of RNA from plant tissue. Anal. Biochem. 163, 16-20.
Odell, J. T.Nagy, F., and Chua, N.-H. (1985). Identification of DNA sequences required for activity of the cauliflower mosaic virus 35S promoter. Nature 313, 810-812.
Piechulla, B., Merforth, N., and Rudolph, B. (1999). Identification of tomato Lhc promoter regions necessary for circadian expression. Plant Mol. Biol. 38, 655-662.,
Saghai-Maroof, M. A., Solimanm, K. M., Jorgensen, R. A., ans Allard, R. W. (1984). Ribosomal DNA spacer length polymorphism in barley: mendelian inheritance, chromosomal location and population dynamics. Proc. Natl. Acad. Sci. USA 81, 8014-8018.
Sambrook , J. , Fritsch, E..F., and Maniatis, T (1989). In Molecular Cloning, A Laboratrory Manual. (Cold Spring Harbor Laboratory Press, New York).
Stahl, D. J., Fischer, R., Dettendorfer, J., Sauerbrey, E., Hain, R., and Nehls, R. (1995). Molecular analysis of the pathogen induced expression of the resveratrol synthase gene in transgenic plants. 4th International Workshop on Pathogenesis related proteins in plants: Biology and biotechnological potential. Kloster Irsee, September3-7.
US 005608150A (Monsanto Company). Fruit specific promoters.
Van der Vossen, E. A. G., Rouppe van der Voort, J. N. A. M., Kanyuka, K., Bendahmane, A., Sandbrink, H., Baulcombe, D. C., Bakker, J., Stiekema, W. J., and Klein-Lankhorst, R. M. (2000). Homologues of a single resistance -gene cluster in potato confer resistance to distinct pathogens: a virus and a nematode. Plant Journal 23(5), 567-576.,
Velten, J., Velten, L., Hain, R., and Schell, J. (1984). Isolation of a dual promoter fragment from Ti plasmid of Agrobacterium tumefaciens. EMBO J. 12, 2723-2730.
WO 94/02619 (Pioneer Hi-Breed International, Inc.) A brassicae regulatory sequence for root-specific or root abundant gene expression.
WO 97/28268 (The Minister of Agriculture and Agri-Food Canada). Promoter from tobacco.
WO 97/27307 (Agritope, Inc). Raspberry promoters for expression of transgenes in plants.
WO 97/32027 (Max-Planck-Gesellschaft zur Förderung der Wissenschaften). Sugarbeet storage-root-tissue-specific regulon.
WO 98/18940 (BASF Aktiengesellschaft). Leaf-specific gene expression in transgenic plants.
WO/98/45460 (Rhone-Poulenc Agro). A sunflower albumin 5'regulatory region for the modification of plant seed lipid composition.
Zhou, D. X. (1999). Regulatory mechanism of plant gene transcription by GT-elements and GT-factors. Trends in Plant Sciences 4, 210-214.

### SEQUENZPROTOKOLL

Application Project
<120> Title : Gewebespezifische Promotoren
   <130> AppFileReference : KWS 0060 PCT/EP/D2
   <140> CurrentappNumber :
   <141> CurrentFilingDate : _-_-_
Earlier Applications
<150> PriorAppNumber : EP00124989.5
   <151> PriorFilingDate : 2000-11-16
Earlier Applications
<150> PriorAppNumber : EP01994666.4
   <151> PriorFilingDate : 2001-11-15
Earlier Applications
<150> PriorAppNumber : PCT/EP01/13214
   <151> PriorFilingDate : 2001-11-15
Sequence
<213> OrganismName : Beta vulgaris
   <400> PreSequenceString : 
ctcgaggatc cttgatttgt tttttgacc aatgagcatt aagagtgatc aagcacatgg 60
atttcaaaat atcaatggtt ggatttttat cattctcaaa tcaatggtta aagttaaata 120
ttctatttc taatttctaa atttataatt ctatactcta gtattgattt gataagcaat 180
tagtaatcta cttaaattcc taaactatta ttaataatat aaattaataa aaattataaa 240
cttagaagtt taattattat ttttaatttc aaacaagttg attgatgtaa atatgattat 300
gaatgacgtg aaacagttaa ggcaaagttg tagtttagta actttaaaaa ttagtagtgt 360
tattataact taatagaaat tataaacttt caaaattaaa ttagtattat tgatttttaa 420
gaaattatta ttctaaattt ataaattcag aaatatgtga acatgttaca tgcggtgatt 480
acggattaat ttaatgctaa catgtaagac gtaacacaat aacagttaac ctactactac 540
tacttgtata ggtcttacat taatacttaa gttattggtt ataaacttat attaatatac 600
ggttatttct tctattaagc gtagatttct tataacctta cttatacaat tatttactat 660
tactcaacta taatttgatt gtattcagtt aagtgaactt atataagtta taacaagcct 720
taatcagtac tagaccttag cttatactat actacatata cgaattttcg cttttcatat 780
tagtatctta ctgataagca ataaagttta ctacgacata gtaacaataa ttaactactt 840
gtttcaattc aattagatga tttgggtttt gaaatgaatt taacttaaag atgattacct 900
tagatagagt attgttatct tctattaagt taattactta acttacatta atacatttgt 960
tgatattatc caatatccat tatcttaaac gaacttacgc catcttgaat tagctaatcg 1020
acttacattg atacatcgat ctaatccaat attaacttgt atcaattcaa attagatgtt 1080
ttgagtttca atatgcaact taacttagag acgattaact taaatgaagc attgttatat 1140
tgaaccaatc tactgagtta tactattcta catctgtttg aattagatgc tttcggttcc 1200
gaaatgaatt caacttaaag atgattaact taagtagagt actgttatta tcttgaatta 1260
aacttagggt actgttaaac tagatgtttt gagtttcaat atgaatttaa cttagagacg 1320
attaacttaa atggagtatt gttatattga accaatctac tgagttccat tgttctacat 1380
ttgttgcaac tcaattagat gctttcagtt ccgaaatgaa ttcaacttaa aaagatgatt 1440
aacttaagta gagtactatt attatattga actagtttat cgacttacat tgatacactc 1500
attcaaataa caacatgttt caatttaagt aaacgttttg agtttcaatt actttatcga 1560
cttacgtcga tacattcatc taatctaata tcaattgtaa tttcttttag tttggctaag 1620
atatgtgagt ttcaaattga atataaattg ttagcgatta acttaaatgg agtaatgtta 1680
tcttgatttc acttatcgat tcacatagat accttgagct aatccataat caacttattt 1740
tacgtcgatt atagatatat taatccattg aactaataca ctaagagtaa gacactaaca 1800
tagatttgtc ttattccaaa tcatatatat taagtaataa agtacacttt aaccgatgtc 1860
actttcattc acgtttttat gaagtacaac tatttttttg aggcaattgt ggaagtgccg 1920
acaacactta tttgttgcat taatttttag ccggacccga aaacacctga ccaaaaccga 1980
catgaagcac aatttttccc gaacaaatac aagagtgtga ttaacttcta cccgaaccga 2040
tgcgattatt gtcgaagtta tgtggttttt tgtcaataac tgaaataaac cgaaacgaaa 2100
ctgaaaataa ccgcaacaaa ttttaactga tatggattca accgttggtt ggtttaactg 2160
tttggttaaa actgatttaa actgcaactg aactgaactg aaaatggaaa gggaaaaaac 2220
tgaattaaac tgaactgaaa agtttgttaa ctgaactgaa ctgttaatta tttcaactaa 2280
tacaaactga actgatatca actgaactgt ttggttataa ccataactga actgttgaac 2340
 
tgaattgaca cctctaattg caacggcaac aatagaactg ttacagtagc ccaatctaca 2400
aaactaaatt gacaagaact gttgcattag atactcctac tacaacggta cgtaataatg 2460
tgttgcacta gtaaatttta gcctttgcct ttgattaaaa atgttgtagt gacacatcac 2520
aataaaatca aatctaatga atatgctata aacaaacaat tgttccaaaa tcaactattc 2580
ttacgaaata ctcttttcga gggacgacat ttttctgaaa caccctaagc gacgttattg 2640
actctaatac atagatgaaa tatcaataaa aagggattta tatgaggtat tctttaattt 2700
ttttttaaga acatatgagg taccatagta ttttaaaaat caatccatgt gcccctaact 2760
tcgtaatgta tccatcatat atatcccttt tttttttagg ggtccatcat atatatcctt 2820
aatgcatgac tatcaaacat tagatatgtc ttaatccccc cctaacaatc aaccaaaatt 2880
ccaaaattgg ttactctatt tgttcctatt acaccttcca ccaatctcaa caaattttgg 2940
cattctggtt aagtactcga aggactactt aacgaaatat ttgatgtttg ttagccatta 3000
aggtttatgt gttggattca ttataaatgg tttgaaaagt ttattatcat ctttattaaa 3060
agttcaatag tacctcatga gaactctcta taaatcaact atattagtta tcaataataa 3120
gatacattcg gattctattt ggcaacacat ttaagtcacc tcaaatgaat aggtgttaaa 3180
agtttaagtc acctaaaata aatagtcatg attaaagtaa gagcttaaat caaatgtaag 3240
ctaaaaaatt gactgaaatt aaattggtat caagtataac atcttaattt aaggtgttaa 3300
aattttcaac taacttatca attgagcttt tttcatcaat ttcacctcta tttcagcttt 3360
gtttttagct tatatttcca aaattgccaa taggtctcat ttatacctaa gatattcaac 3420
aatcctggct ctcttcctgc tcgttcattc caaatttccc aaatgtaatc agattaaagc 3480
agtagtgatc ggatcattgc agacttttcg gtagaataac tgaataagtg tactgagata 3540
gtgtcaaaat actgttattt aagtttctta taagtactaa aatatgaatt agttcaacat 3600
catcttatat tgtcggccaa taagattgcc cactgtataa taatataaaa caaaatcaca 3660
gaggtgatat cgccttaaaa aaaaaaaatc atagatgtga attaatgtaa ccgagcataa 3720
atcaactgtt tccttttttt ttaaaagaga aatcaactgt ttcctagaaa caattgaata 3780
ttatatggtg gtttattgtt tcaaatttca gaaaaacaaa aaaaaaaaag cctttgctac 3840
ttacttacat agttgcatct atataaaggg cacctagccc aaccatttta cttacctaaa 3900
ctgtctgtgt ttgcaccact ctcatctaaa aaatctgtga aagtgagcgt ttcaaaattt 3960
tcaaaagatg cacataacag gtaaattaga agttgaagtt gatatcaatt gtcatggtga 4020
tattttccat gaaattttta gcaccaggcc acatgatgtc tccaccatgt cacctgagaa 4080
tatccatgg 4089
<212> Type : DNA
   <211> Length : 4089 SequenceName : 1 SequenceDescription :
Sequence
<213> OrganismName : Beta vulgaris
   <400> PresequenceString : 
tatgatattg tcatgctgca tgcatcctca tcgtaaacaa attactccct tcgttttaaa 60
atctagttca tgtttctttt tgcacagtat taaagacata cttttgacct accacctacg 120
atatcttctt atttcatatt aataaaaaat tttaaaaagt tgatattata aatttacaca 180
ttgtggtgaa tctaacaaca cccccacatg aatatattat tttatgtaca tcagtgagag 240
tatttggtca aaaaaaataa agtttgactt ccaaaataga aacatgaact atattttgaa 300
acggccgagt gagtacttaa ttgttgttct acccatcaaa attagaaaaa aggtttttgc 360
tagtaaggaa ttctcttttt gttcgtttag gaaaagtgat aaatgataac gtctttttaa 420
tttaacgcgt gattgtgatc gtggtcctta cctgggttaa attaacatgt tcagactaca 480
ctcgtctttg ctagtggaaa ataaattgca tgttaagacg atgtttagtt ggaatgaaaa 540
acacttctct tagtatattt ggtaaaggtt gtaaaatatg gtgtgaaata aaaatctaag 600
tggagaaaca ttcaaggaaa gaatctcata atttcgttca tatccattta cttcctcttt 660
ctccatcttt tttctcttct ttctaaactt cccttcccat cactaaccaa ataatagaaa 720
gctaattaac aagtatgtat tcttttggca aaaattatta aaaaaaacag tttaatttaa 780
tataaaagta gacctagtaa ctaaataaaa aaaaaaaaat aaattaaact agtcaccaaa 840
aaaaattaaa ctagtcatta ttgggctaat tttcttttta atattgcgtt taccattgtc 900
taattttaat ttaaaagtag gcggtctctt acaagataaa ctccttgagc aaatgttttt 960
ccttgagcaa atgtttttca tagaaaatct cattttctat acaatcaaac gcgcaaactc 1020
aacctaatta aagatttcgt tcagtatcac tatcaacgtt cagttttcta attggattat 1080
gattttacat atacactcat ctttatctaa aagatattaa ttgcataaac agtaaaaata 1140
taatatacaa tgataaaaaa aaattcgaag tatcactatc atctaaagag tgtattcatc 1200
atacatctgg gtgcaccatg tgcatggtac acacacgatt attttttgct catttgaagc 1260
tctacctatt ttttaattat gctcagttgg aagtttcaaa tgagcaatga gtaaaaaaaa 1320
aaccttgaat gtaccatact catgatatat ccgagtgtac cataaaaatt cccatcatct 1380
caatgtagta ctataaattt tttaataaag tccaaatatt aatagaaggg aacacatgaa 1440
aagagtagaa ttgaattagg tacataaaaa gttgcattca tatctttttc tccttggcat 1500
aacaatcttg acaatctaat ttccctattt aaatccacta acttcataga cttcatgccc 1560
caacaacagg aaagtggtct cctatccctt ttagtatcat ttcataattt caattttaaa 1620
ttaaaagggc aattagatgg cattcttttg cattattggt attataaatt aatcatcgtg 1680
ttatctttat ctagctagaa ataccacttc caagtaaagt ttatgtcttt tgcatcatac 1740
 
taacaataag ccacaccatt atttcttgta cttcccttga agaaaacaaa gagaattaat 1800
agaaactaag tgagagaaag agatatagag atagtttcac atgaacatgg cttcattgcc 1860
actacaaaac ttggtcttct ttttatcatt atttctgctt cacttatatc aacttcata 1919
<212> Type : DNA
   <211> Length : 1919 SequenceName : 2 SequenceDescription :
Sequence
<213> OrganismName : Beta vulgaris
   <400> PreSequenceString :

tctaggccta aatggccatt taggtgacac tatagaagag ctcgaggatc aacattcttc 60
tgaatcgttc ataaatacga atcttacaat atcttgtatg aatatatact ataccttttt 120
atagattgaa ataaaactcc aggcgcctta tttcagattt ggagagcata atatcaacaa 180
agcaaaacat attcaaccat tatacaaacc gaaaagtttt tgatgtcact taaccaaaat 240
ttcaaaaatg atacatatgt gcagcctaag ttctatattt cacctaccta gttctataca 300
ctagcaatca taatggtcaa ttaatagtac tctcctccaa aacaacattg agtcaattag 360
agactatcgg aataggagcg aacaactaac aaatatcaac taaataatat agcggaaatc 420
tatttgtttg tcgtaataat ttttttcaat tatttttgtt tgaatattta ctcgtgtaat 480
agatgtcaat taatttagcg gtatgatttt ctaaaaagat gagctaacct aaaacttaat 540
attgtctata gcacttgcct tatggctcct aagtcctaac caccataagt tttacttggg 600
cataagatga ctcaaatcct accaatcttt catcattata actaagggct cggctatatc 660
ttcccagata tatcatggta agaaaacaca acaacctacc acctgtcaac cactagatgg 720
atagctaaga ttccacttag gaaaaatctt ccaacatggc atttaaattg gacccatatg 780
atctattaga gccaacagaa acaaaggaat agatatatgg atatggcatt agataaggat 840
agtgaaaaac acttctcatt ggctaatctt taactctcaa acccaatcta gcccttgatt 900
tccctacaac aacaaaaaaa tccaactcac taaagttcca aataactcct aaataaacaa 960
gagccacaag acttgtattg aaatgaaaat acaagggaga gcgttacact agacaaagcc 1020
atcacaactt tgttaacact cttccttctt aatcatttcc cttcaattaa atttcttagt 1080
tgaaaccaaa atataagatg gcagcagtga caacacaggc ttcgattgcc ggattccggc 1140
catgcgca 1148

<212> Type : DNA
   <211> Length : 1148 SequenceName : 3 SequenceDescription :
Sequence
<213> OrganismName : Beta vulgaris
   <400> PreSequenceString : 
ctgcagacag cggggaatgt caggtgctgg cgcgatggga cacaccgaag gtggtgaagg 60
gcgtgagttt cctgctccgt ctgaccgtaa cagcggacga cggcagtgag cggctggtca 120
gcacggcccg gacgacggaa accacatacc gcttcacgca actggcgctg gggaactaca 180
ggctgacagt ccgggcggta aatgcgtggg ggcagcaggg cgatccggcg tcggtatcgt 240
tccggattgc cgcaccggca gcaccgtcga ggattgagct gacgccgggc tattttcaga 300
taaccgccac gccgcatctt gccgtttatg acccgacggt acagtttgag ttctggttct 360
cggaaaagca gattgcggat atcagacagg ttgaaaccag cacgcgttat cttggtacgg 420
cgctgtactg gatagccgcc agtatcaata tcaaaccggg ccatgattat tacttttata 480
tccgcagtgt gaacaccgtt ggcaaatcgg cattcgtgga ggccgtcggt cgggcgagcg 540
atgatgcgga aggttacctg gattttttca aaggcaagat aaccgaatcc catctcggca 600
aggagctgct ggaaaaagtc gagctgacgg aggataacgc cagcagactg gaggagtttt 660
cgaaagagtg gaaggatgcc agtgataagt ggaatgccat gtgggctgtc aaaattgagc 720
agaccaaaga cggcaaacat tatgtcgcgg gtattggcct cagcatggag gacacggagg 780
aaggcaaact gagccagttt ctggttgccg ccaatcgtat cgcatttatt gacccggcaa 840
acgggaatga aacgccgatg tttgtggcgc agggcaacca gatattcatg aacgacgtgt 900
tcctgaagcg cctgacggcc cccaccatta ccagcggcgg caatcctccg gcctttcccc 960
tgacaccgga cggaaagctg accgctaaaa atgcggatat cagtggcagt gtgaatgcga 1020
actccgggac gctcagtaat gtgacgatag ctgaaaactg tacgataaac ggtacgctga 1080
gggcggaaaa aatcgtcggg gacattgtaa aggcggcgag cgcggctttt ccgcgccagc 1140
gtgaaagcag tgtggactgg ccgtcaggta cccgtactgt caccgtgacc gatgaccatc 1200
cttttgatcg ccagatagtg gtgcttccgc tgacgtttcg cggaagtaag cgtactgtca 1260
gcggcaggac aacgtattcg atgtgttatc tgaaagtact gatgaacggt gcggtgattt 1320
atgatggcgc ggcgaacgag gcggtacagg tgttctcccg tattgttgac atgccagcgg 1380
gtcggggaaa cgtgatcctg acgttcacgc ttacgtccac acggcattcg gcagatattc 1440
cgccgtatac gtttgccagc gatgtgcagg ttatggtgat taagaaacag gcgctgggca 1500
 
tcagcgtggt ctgagtgtgt tacagaggtt cgtccgggaa cgggcgtttt attataaaac 1560
agtgagaggt gaacgatgcg taatgtgtgt attgccgttg ctgtctttgc cgcacttgcg 1620
gtgacagtca ctccggcccg tgcggaaggt ggacatggta cgtttacggt gggctatttt 1680
caagtgaaac cgggtacatt gccgtcgttg tcgggcgggg ataccggtgt gagtcatctg 1740
aaagggatta acgtgaagta ccgttatgag ctgacggaca gtgtgggggt gatggcttcc 1800
ctggggttcg ccgcgtcgaa aaagagcagc acagtgatga ccggggagga tacgtttcac 1860
tatgagagcc tgcgtggacg ttatgtgagc gtgatggccg gaccggtttt acaaatcagt 1920
aagcaggtca gtgcgtacgc catggccgga gtggctcaca gtcggtggtc cggcagtaca 1980
atggattgcc gtaagacgga aatcactccc gggtatatga aagagacgac cactgccagg 2040
gacgaaagtg caatgcggca tacctcagtg gcgtggagtg caggtataca gattaatccg 2100
gcagcgtccg tcgttgttga tattgcttat gaaggctccg gcagtggcga ctggcgtact 2160
gacggattca tcgttggggt cggttataaa ttctgattag ccaggtaaca cagtgttatg 2220
acagcccgcc ggaaccggtg ggcttttttg tggggtgaat atggcagtaa agatttcagg 2280
agtcctgaaa gacggcacag gaaaaccggt acagaactgc accattcagc tgaaagccag 2340
acgtaacagc accacggtgg tggtgaacac ggtgggctca gagaatccgg atgaagcctg 2400
cttttttata ctaagttggc attataaaaa agcattgctt atcaatttgt tgcaacgaac 2460
aggtcactat cagtcaaaat aaaatcatta tttgatttca attttgtccc actccctgcc 2520
tctgtcatca cgatactgtg atgccatggt gtccgactta tgcccgagaa gatgttgagc 2580
aaacttatcg cttatctgct tctcatagag tcttgcagac aaactgcgca actcgtgaaa 2640
ggtaggcgga tctgggtcga ctctaggcct cactggccta atacgactca ctatagggag 2700
ctcgaggatc tattagagcc aacagaaaca aaggaataga tatatggata tggcattaga 2760
taaggatagt gaaaaacact tctcattggc taatctttaa ctctcaaacc caatctaacc 2820
cttgatttcc ctacaacaac aaaaaaatcc aactcactaa agttccaaat aactcctaaa 2880
taaacaagag ccacaagact tgtattgaaa tgaaaataca aggaagagcg ttacactaga 2940
caaagccatc acaactttgt taacactctt ccttcttatt tccattccaa aaaatataat 3000
ggcagcagtg acaacacagg cttcgattgc cggattccgg ccatgcgca 3049
<212> Type : DNA
   <211> Length : 3049 SequenceName : 4 SequenceDescription :
Sequence
<213> OrganismName : künstliche Sequenz
   <400> PreSequenceString :
   ctgcttctaa tagagtcttg ctgcagacaa actgcgcaac 40

   <212> Type : DNA
   <211> Length : 40 SequenceName : 5 SequenceDescription :
Sequence
<213> OrganismName : künstliche Sequenz
   <400> PreSequenceString :
   tgaacactcg tccgagaata acgagtggat ctgggtc 37
   <212> Type : DNA

   <211> Length : 37 SequenceName : 6 SequenceDescription :
Sequence
<213> OrganismName : künstliche Sequenz
   <400> PreSequenceString :
   aggttatcaa aaggcccctt tccagtca 28
   <212> Type : DNA
   <211> Length : 28 SequenceName : 7 SequenceDescription :
Sequence
<213> OrganismName : künstliche Sequenz
   <400> PreSequenceString :
   gtttgtgcaa gccgagctgg tgaacgcc 28
   <212> Type : DNA
   <211> Length : 28 SequenceName : 8 SequenceDescription :
Sequence
<213> OrganismName : künstliche Sequenz
   <400> PreSequenceString :
   gtggagaggc tattcggta 19
   <212> Type : DNA
   <211> Length : 19 SequenceName : 9 SequenceDescription :
Sequence
<213> OrganismName : künstliche Sequenz
   <400> PreSequenceString :
   ccaccatgat attcggcaag 20
   <212> Type : DNA
   <211> Length : 20 SequenceName : 10 SequenceDescription :
Sequence
<213> OrgamsmName : künstliche Sequenz
   <400> PreSequenceString :

   caannnnatc 10
   <212> Type : DNA
   <211> Length : 10 SequenceName : 11 SequenceDescription :
Sequence
<213> OrganismName : künstliche Sequenz
   <400> PreSequencestring :
   caagcttatg catgcggccg catctagagg gcccggatcc aaatg 45
   <212> Type : DNA

   <211> Length : 45 SequenceName : 12 SequenceDescription :
Sequence
<213> OrganismName :
   <400> PreSequenceString :
   atggcagcag tgacaacaca ggcttcgatt gccggattcc ggccatgccg gatccaaatg 60
<212> Type : DNA
   <211> Length : 60 SequenceName : 13 SequenceDescription :
Sequence
<213> OrganismName : künstliche Sequenz
   <400> PreSequenceString : 
atggcagcag tgacaacaca ggcttcgatt gccggattcc ggccatgccg gatccccggg 60
tacggt 66
<212> Type : DNA
<211> Length : 66 SequenceName : 14 SequenceDescription :

## Patentansprüche

1. In oberirdischen Pflanzenteilen spezifisch aktiver Promotor, umfassend eine Nukleotidsequenz der SEQ ID NO: 3 oder eine zu der Nukleotidsequenz der SEQ ID NO: 3 komplementäre Nukleotidsequenz oder eine Nukleotidsequenz, die mit der Nukleotidsequenz der SEQ ID NO: 3 oder einer zu der Nukleotldsequenz der SEQ ID NO: 3 komplementären Nukleotidsequenz unter stringenten Bedingungen hybridisiert.

2. Vektor oder mobiles genetisches Element, enthaltend einen Promotor nach Anspruch 1.

3. Eukaryotische oder prokaryotische Wirtszelle, die ein Genkonstrukt, das den Promotor nach Anspruch 1 aufweist, als Transgen oder den Vektor oder das mobile genetische Element nach Anspruch 2 enthält.

4. Pflanze oder deren Teile, die ein Genkonstrukt, das den Promotor nach Anspruch 1 aufweist, als Transgen enthalten.

5. Pflanze nach Anspruch 5, **dadurch gekennzeichnet, daß** die Pflanze *Beta vulgaris* ist.

6. Samen von Pflanzen nach Anspruch 4 oder 5, enthaltend ein Genkonstrukt, das den Promotor nach Anspruch 1 aufweist, als Transgen.

7. Verwendung eines Promotors nach Anspruch 1 zu einem oder mehreren der folgenden Zwecke, insbesondere bei Pflanzen:
a. Veränderung des Kohlenhydratmetabolismus
b. Vermeidung von Speichersubstanzverlusten
c. Expression eines Invertaseinhibitors
d. Expression einer Fructosyltransferase
e. Expression einer Levansucrase
f. Expression von Genen codierend für Transporterproteine für N-Verbindungen
g. Ausprägung von Merkmalen, die die Resistenz/Toleranz gegenüber Pathogenen erhöhen

8. Transgene Pflanze, die eine Expression von mindestens einem übertragenen Gen in spezifischen Pflanzenteilen ermöglicht, wobei die Expression in oberirdischen und Chloroplasten enthaltenden Pflanzentellen, wie Blättern, und nicht in unterirdischen Organen der Pflanze erfolgt, und wobei die Pflanze ein Genkonstrukt, das den Promotor nach Anspruch 1 operativ verbunden mit dem übertragenen Gen aufweist, als Transgen enthält.

9. Transgene Pflanze nach Anspruch 8, **dadurch gekennzeichnet, daß** es sich bei den unterirdischen Organen um Wurzeln handelt.

10. Transgene Pflanze nach Anspruch 8 oder 9, die der Gattung *Beta vulgaris* angehört.

11. Transgene Pflanze nach einem der Ansprüche 8 bis 10 mit einer oder mehreren der folgenden Eigenschaften:
a. Veränderung des Kohlenhydratmetabolismus
b. Vermeidung von Speichersubstanzverlusten
c. Expression eines Invertaseinhibitors
d. Expression einer Fructosyltransferase
e. Expression einer Levansucrase
f. Expression von Genen codierend für Transporterproteine für N-Verbindungen
g. Ausprägung von Merkmalen, die die Resistenz/Toleranz gegenüber Pathogenen erhöhen

## Claims

1. In above-ground plant parts specifically active promoter comprising a nucleotide sequence of SEQ ID NO: 3 or a nucleotide sequence complementary to the nucleotide sequence of SEQ ID NO: 3 or a nucleotide sequence, which hybridizes with the nucleotide sequence of SEQ ID NO: 3 or with a nucleotide sequence, which is complementary to the nucleotide sequence of SEQ ID NO: 3, under stringent conditions.

2. Vector or mobile genetic element comprising a promoter according to claim 1.

3. Eukaryotic or prokaryotic host cell which comprises a gene construct containing the promoter according to claim 1 as transgene or which comprises the vector or mobile genetic element according to claim 2.

4. Plant or parts thereof, which comprise a gene construct containing the promoter according to claim 1 as transgene.

5. Plant according to claim 5, **characterized in that** the plant is *Beta vulgaris.*

6. Seeds of plants according to claim 4 or 5 comprising a gene construct containing the promoter according to claim 1 as transgene.

7. Use of a promoter according to claim 1 for one or multiple of the following purposes especially in plants:
a. alteration of the carbohydrate metabolism
b. avoidance of loss of storage substance
c. expression of an invertase inhibitor
d. expression of a fructosyl transferase
e. expression of a levan sucrase
f. expression of genes coding for transporter proteins for N-compounds
g. development of traits, which increase the resistance/tolerance towards pathogens.

8. Transgenic plant, which allows the expression of at least one transferred gene in specific parts of the plant, wherein the expression occurs in above-ground chloroplast containing plant parts as leaves and not in belowground organs of the plant and wherein the plant comprises a gene construct containing the promoter according to claim 1 operably linked to the transferred gene as transgene.

9. Transgenic plant according to claim 8, **characterized in that** the belowground organs are roots.

10. Transgenic plant according to claim 8 or 9, which belongs to genus *Beta vulgaris*.

11. Transgenic plant according to one of claims 8 to 10 with one or multiple of the following characteristics:
a. alteration of the carbohydrate metabolism
b. avoidance of loss of storage substance
c. expression of an invertase inhibitor
d. expression of a fructosyl transferase
e. expression of a levan sucrase
f. expression of genes coding for transporter proteins for N-compounds
g. development of traits, which increase the resistance/tolerance towards pathogens.

## Revendications

1. Promoteur pour expression dans les organes situés au-dessus du sol de la plante comprenant une séquence nucléotide de la SEQ ID NO: 3 ou une séquence nucléotide complémentaire à la séquence nucléotide de la SEQ ID NO: 3 ou une séquence nucléotide qui s'hybride avec la séquence nucléotide de la SEQ ID NO: 3 ou à une séquence nucléotide complémentaire à la séquence nucléotide de la SEQ ID NO: 3 dans des conditions astringentes.

2. Vecteur ou élément génétique mobile, contenant un promoteur selon la revendication 1.

3. Cellule hôte eucaryote ou procaryote contenant, en tant que transgène, un produit de recombinaison génétique comportant le promoteur selon la revendication 1 ou contenant le vecteur ou l'élément génétique mobile selon la revendication 2.

4. Plante ou ses parties contenant, en tant que transgène, un produit de recombinaison génétique contenant le promoteur selon la revendication 1.

5. Plante selon la revendication 4, **caractérisée en ce que** la plante est la beta vulgaris.

6. Graines de plantes selon les revendications 4 ou 5, contenant, en tant que transgène, un produit de recombinaison génétique comportant le promoteur selon la revendication 1.

7. Utilisation d'un promoteur selon la revendication 1 à une ou plusieurs des fins suivantes en particulier dans des plantes :
a. Modification du métabolisme des glucides
b. Evitement des pertes de réserves de substances
c. Expression d'un inhibiteur d'invertase
d. Expression d'une fructosyl-transferase
e. Expression d'une lévane-sucrase
f. Expression de gènes codante pour les protéines transporteuses pour composés N
g. Inculcation de caractéristiques augmentant la résistance/tolérance aux pathogènes

8. Plante transgénique permettant une expression d'au moins un gène transféré dans des parties spécifiques de la plante, ladite expression ayant lieu dans des parties de plante aériennes et contenant des chloroplastes, telles que les feuilles, et non dans les organes souterrains de la plante, et ladite plante contenant, en tant que transgène, un produit de recombinaison génétique comportant ledit promoteur selon la revendication 1 relié opératoirement au gène transféré.

9. Plante transgénique selon la revendication 8, **caractérisée en ce que** les organes souterrains sont des racines.

10. Plante transgénique selon la revendication 8 ou 9, appartenant à la variété beta vulgaris.

11. Plante transgénique selon une des revendications 8 à 10, dotée d'une ou plusieurs des propriétés suivantes :
a. Modification du métabolisme des glucides
b. Evitement des pertes de réserves de substances
c. Expression d'un inhibiteur d'invertase
d. Expression d'une fructosyl-transferase
e. Expression d'une lévane-sucrase
f. Expression de gènes codante pour les protéines transporteuses pour composés N
g. Inculcation de caractéristiques augmentant la résistance/tolérance aux pathogènes
